(19) Europäisches Patentamt
European Patent Office
Office européen des brevets

(11) **EP 3 558 901 B1**

(12) # EUROPÄISCHE PATENTSCHRIFT

(45) Veröffentlichungstag und Bekanntmachung des Hinweises auf die Patenterteilung:
**15.07.2020 Patentblatt 2020/29**

(21) Anmeldenummer: **17817769.7**

(22) Anmeldetag: **22.12.2017**

(51) Int Cl.:
*C07B 43/00* (2006.01)     *C07C 291/04* (2006.01)
*C07D 295/24* (2006.01)

(86) Internationale Anmeldenummer:
**PCT/EP2017/084391**

(87) Internationale Veröffentlichungsnummer:
**WO 2018/115443 (28.06.2018 Gazette 2018/26)**

(54) **HERSTELLUNG EINES AMINOXIDS DURCH OXIDATION EINES TERTIÄREN AMINS**

PRODUCTION OF AN AMINE OXIDE BY OXIDATION OF A TERTIARY AMINE

PRODUCTION D'UN AMINOXYDE PAR OXYDATION D'UNE AMINE TERTIAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2016 EP 16206623**

(43) Veröffentlichungstag der Anmeldung:
**30.10.2019 Patentblatt 2019/44**

(73) Patentinhaber: **Aurotec GmbH**
**4844 Regau (AT)**

(72) Erfinder:
• ZIKELI, Stefan
**4844 Regau (AT)**

• KITZLER, Hannes
**4860 Lenzing (AT)**
• FOSODEDER, Verena
**4872 Neukirchen an der Vöckla (AT)**
• BAUMEISTER, Tobias
**68163 Mannheim (DE)**

(74) Vertreter: **Sonn & Partner Patentanwälte**
**Riemergasse 14**
**1010 Wien (AT)**

(56) Entgegenhaltungen:
WO-A1-2010/055034     WO-A1-2013/030400
DE-A1- 3 618 352      DE-B- 1 221 645
US-A- 3 447 939

**Beschreibung**

Die vorliegende Erfindung betrifft das Gebiet der Oxidation von tertiären Aminen zu Aminoxiden.

**Hintergrund der Erfindung**

Zellulose kann in wässrigen Lösungen von Aminoxiden, z.B. von Lösungen von N-Methyl-Morpholin-N-oxid (NMMO oder NMMNO), gelöst werden, um aus der erhaltenen Spinnlösung Spinnprodukte, wie zum Beispiel Filamente, Stapelfasern, Folien, etc. herzustellen (WO 2013/030400, US 2,179,181, US 3,447,939). Daher besteht ein wachsender Bedarf an Aminoxiden wie NMMO. Weitere Anwendungen von tertiären Aminoxiden sind oberflächenaktive Mittel, Bakteriostatika, Shampoos und Waschmittel.

Die DE 2557456 A1 beschreibt ein Verfahren zur Herstellung von Aminoxiden, wobei ein tertiäres Amin mit Wasserstoffperoxid in Gegenwart von Wasser umgesetzt wird. Bei einer Aminoxidkonzentration von über 30% steigt die Viskosität des Produktes stark an, wodurch die weitere Verarbeitung erschwert wird. Es wurde erkannt, dass bei Aminoxidkonzentration über 60% ein Viksositätsminimum erreicht wird.

Die US 3,447,939 beschreibt die diskontinuierlich betriebene Batch Herstellung von N-Methyl-Morpholin-N-Oxide (NMMNO), wobei das Edukt N-Methylmorpholin (NMM) in Gegenwart von Wasser und 35%igem Wasserstoffperoxid, bei den gewählten Reaktionstemperaturen von 67-72°C über einen Zeitraum von 4-5 Stunden in das entsprechende tertiäre Aminoxid NMMNO umgesetzt wird. Die Zersetzung des überschüssigen Wasserstoffperoxides erfolgt enzymatisch mit Katalase. Das gebildete Reaktionsprodukt wurde durch azeotrope Benzol-Wasser Destillation und Umkristallisation gereinigt.

In der DD 246997 A1 wird ein ähnliches Batch-Verfahren beschrieben, in dem Wasserstoffperoxid (45-50%) über einen Sprühteller oder eine Einspritzvorrichtung in feinverteilter Form unter starkem Rühren in das Edukt NMM eingebracht wird.

In dem Verfahren nach DE 3618352 A1 wird nach einer Oxidation mittels Wasserstoffperoxid über 4-6 Stunden verbleibendes Peroxid durch Katalase abgebaut.

EP 0 307 184 A2 greift mit Bezug auf das US Patent 4,247,480 das Problem der Nitrosaminbildung während der Umsetzung von tertiärem Amin zum entsprechenden Aminoxid auf und schlägt vor, dass bei Einsatz von Kohlendioxid Atmosphäre (0,03-3 MPa) die Reaktion bei niedrigen Temperaturen <45°C und Normaldruck durchgeführt werden soll.

Da die Nitrosaminbildung auch von ubiquitär vorhandenen Stickoxiden der Luft beeinflusst werden kann schlägt US 4,994,614 A vor, den unbefüllten Raum des Reaktionskessels mit Kohlendioxid zu überlagern, damit die Stickoxide der Luft ferngehalten werden. Solch eine Spül- und Überlagerungsmaßnahme ist sehr aufwändig, da die Reaktionsbehälter vor der Zugabe der Edukte, während der Reaktion, nach Anschluss der Reaktion und während der Behälterentleerung mit Kohlendioxid gespült bzw. überlagert werden müssen.

US 5,216,154 A schlägt zur Reduzierung sich bildender Nitrosamine vor, dass die Umsetzung vom tertiären N-Methylmorpholin (NMM) zu NMMNO im Rührkessel, unter einer bestimmten Eduktgemischzusammensetzung und speziellen Verfahrensparametern durchgeführt werden soll.

DE 12 21 645 B beschreibt eine Oxidation von tertiären Aminen beschrieben im diskontinuierlichen Prozess. Beiläufig wird eine kontinuierliche Oxidierung bei turbulenter Prozessführung erwähnt.

Es ist ein Ziel der vorliegenden Erfindung einen skalierbaren Prozess zur Herstellung von Aminoxiden zur Verfügung zu stellen, der eine hohe Ausbeute, geringe bis keine Neben- und Abbauprodukte und eine effiziente Umwandlung ermöglicht.

**Zusammenfassung der Erfindung**

Die vorliegende Erfindung betrifft ein Verfahren zur Herstellung eines Aminoxids durch Oxidation eines tertiären Amins in einem Reaktor mit einem oder mehreren länglichen Hohlkörper als Reaktionsbereich, wie z.B. ein Rohr, wobei der Reaktor vorzugsweise ein Reaktionsrohr ist, unter kontinuierlicher Einführung von tertiärem Amin in einem Reaktionsfluid und Ausführung von Aminoxid. Dazu wurden geeignete Prozessparameter, insbesondere ein geeignetes Oberfläche-zu-Volumenverhältnis bzw. eine passende Strömungsgeschwindigkeit mit entsprechenden Oberflächen/Volumenbelastungen im kontinuierlichen Prozess gewählt. Insbesondere ist im Hohlkörper i) ein Oberfläche zu Volumenverhältnis von 0,5 $m^2/m^3$ oder mehr - zumindest auf einer Länge des Hohlkörpers, in der das Amin zu 50% oxidiert wird, ii) eine spezifische Oberflächenbelastung von 1 $l/m^2h$ bis 40 $l/m^2h$, und/oder iii) eine spezifische Volumenbelastung von 1.000 $l/m^3h$ bis 30.000 $l/m^3h$, vorhanden. Diese drei Kennzeichen i), ii) und iii) betreffen im Grunde alternative Definitionen, welche das erfindungsgemäße Verfahren beschreiben können. In manchen Ausführungsformen treffen alle Parameter i), ii) und iii) zu. Die folgende ausführliche Beschreibung und insbesondere die vorzugsweisen Ausführungsformen betreffen alle drei Parameter i), ii) und iii) gleichermaßen. Weiterhin wird im Hohlkörper das Reaktionsfluid mit laminarer Strömung geführt.

Alle erfindungsgemäßen Ausführungsformen und Aspekte sind miteinander kombinierbar, und wurden dies auch, wie in den experimentellen Beispielen gezeigt wurde.

## Kurzbeschreibung der Figuren

Figur 1 zeigt den Zusammenhang von Verweilzeit im Hohlkörper und Umsatz für den Fall der Oxidation von NMM.
Figur 2 zeigt einen Mikroreaktor (1) mit Zufuhr der Edukte (3,4) über die Pumpen (5,6) und Ableitung nach dem Druckhalteventil (11) zum Produktbehälter (12).
Figur 3 zeigt den Ausbeuteverlauf bei unterschiedlicher $CO_2$-Beimischung.
Figur 4 zeigt den Umsatzverlauf bei unterschiedlicher $CO_2$-Beimischung.
Figur 5 zeigt Umsätze in einem Reaktor aus Hastelloy® C-22.
Figur 6 zeigt die Stabilisierung des Wasserstoffperoxids mit Phosphorsäure zur Reaktionsverbesserung.
Figur 7 zeigt den Einfluss der $CO_2$ Konzentration auf die Reaktionsgeschwindigkeit.
Figur 8 zeigt den Einfluss der Temperatur auf den Reaktionsfortschritt, Umsatz-Ausbeuteverlauf bei 30 Gew.% $H_2O_2$, 1 Gew.% $CO_2$, 0,1 Gew.% $H_3PO_4$.
Figur 9 zeigt den Einfluss der Konzentration des Wasserstoffperoxids auf die Produktionsrate.
Figur 10 zeigt Ergebnisse mit einem Großvolumenreaktor.
Figur 11 zeigt einen Mikroreaktor analog zu Fig. 2 mit zusätzlicher Zuleitung für ein 3. Edukt (z.B. $CO_2$).
Figur 12 zeigt Ergebnisse zur separaten $CO_2$-2ufuhr.

## Detaillierte Beschreibung der Erfindung

Die Erfindung betrifft ein Verfahren zur Herstellung eines Aminoxids durch Oxidation eines tertiären Amins in einem Reaktor unter kontinuierlicher Einführung von tertiärem Amin in einem Reaktionsfluid und Ausführung von Aminoxid, wobei ein geeignetes Oberfläche-zu-Volumenverhältnis bzw. eine passende Strömungsgeschwindigkeit mit entsprechenden Oberflächen/Volumenbelastungen im kontinuierlichen Prozess gewählt werden. Der kontinuierliche Prozess wird in einem länglichen Hohlkörper, mit einem länglichen Bereich zwischen einem Einlass und Auslass durchgeführt. Die erfindungsgemäßen Parameter und baulichen Merkmale finden sich zwischen Ein- und Auslass, also in der Reaktions- oder Reaktorzone. Der Reaktor ist insbesondere ein milli- oder mikrostrukturierter Reaktor oder Apparat. Der Hohlköper ist insbesondere bevorzugt ein Rohr. Insbesondere ist im Hohlkörper i) ein Oberfläche zu Volumenverhältnis von 0,5 $m^2/m^3$ oder mehr - zumindest auf einer Länge des Hohlkörpers, in der das Amin zu 50% oxidiert wird, ii) eine spezifische Oberflächenbelastung von 1 $l/m^2h$ bis 40 $l/m^2h$, und/oder iii) eine spezifische Volumenbelastung von 1.000 $l/m^3h$ bis 30.000 $l/m^3h$, vorhanden.

Bisher werden tertiäre Aminoxide für kleinere Mengen im Labor oder für größere Mengen in Chargenprozessen hergestellt. Es ist ein Ziel für Großanlagen ein möglichst kontinuierliches Verfahren zum Einsatz kommen zu lassen, da dieses eine effizientere Reaktionsführung zulässt und höhere Produktivitäten durch geringere Stillstandszeiten erreicht werden, da dadurch unnötige Reinigungsschritte vor dem nächsten Chargenprozess vermieden werden. Zudem hat sich gezeigt, dass Schutzgase ($CO_2$) in Chargenprozessen notwendig sind und der Reaktionsflüssigkeit überlagert werden müssen. Im erfindungsgemäßen milli- oder mikrostrukturierten Apparat erübrigt sich dieses Problem, da das Schutzgas direkt in die Rohre eingebracht werden kann, ohne, dass ein Gasbereich entsteht oder schädlich wäre. Erfindungsgemäß lassen sich sowohl Druck als auch Temperatur besser kontrollieren.

Zudem ist es in einem kontinuierlichen Reaktor möglich, das durchgehend bewegte Reaktionsfluid in einzelne Bereiche zu unterteilen, in denen die Reaktion in unterschiedlichen Fortschrittsstadien ist. Diese Bereiche können unterschiedlich hinsichtlich Druck und/oder Temperatur bzw. Temperierung behandelt werden. Solche Bereiche sind insbesondere der erste Bereich, in dem bis zu 50% (mol.-%) des tertiären Amins oxidiert werden (schnelle Reaktion, hohe Exothermie) und der zweite Bereich, in dem der Rest des tertiären Amins (bis zur gewünschten Ausbeute) oxidiert wird (langsamere Reaktion, geringere Exothermie).

"Kontinuierliche" Reaktion oder Prozessführung bedeutet, dass laufend während der Reaktion Ausgangsstoff, also Fluid mit dem tertiären Amin zugeführt wird, und dieses daraufhin laufend im Reaktor oxidiert wird. Laufend bedeutet nun nicht, dass der Prozess nicht gestoppt werden darf, sondern bloß, dass während der Herstellung von Aminoxid die Edukte mehr oder weniger gleichmäßig zugeführt werden. Vorzugsweise ist die kontinuierliche Zuführung während der Oxidationsreaktion ununterbrochen. Alternativ, aber weniger bevorzugt, kann auch eine absatzweise und/oder periodische Zufur gewählt werden. Gesagtes zur Zuführung gilt ebenso für eine Abfuhr des Produkts, also des Aminoxids.

Der Reaktor ist üblicherweise ein milli- oder mikrostrukturierter Apparat und/oder ein Reaktor mit einem länglichen Hohlkörper als Reaktionskörper, vorzugsweise ein rohrartiger Mikroreaktor. Ein geeigneter Mikroreaktor ist beispielsweise in der WO 2010/055034 (unter Bezugnahme hierin aufgenommen) offenbart. Ein Reaktor kann ein oder mehrere parallele längliche Hohlkörper enthalten, in denen die Reaktion durchgeführt wird. Der Definitionen des Begriffs "Hohlkörper" werden verwendete um die Gesamtheit der Hohlkörper für die Reaktion zu beschreiben - sofern nicht zu anderen Zwecken vorgesehen, wie dem Vorheizen. In den Hohlkörpern wird das Reaktionsfluid befördert, welches durch den Einlass des Ausgangsstoffes (bzw. der Ausgangsstoffe) gesteuert wird. Das Fluid wird als Reaktionsfluid bezeichnet, sobald alle zur Oxidation nötigen Bedingungen bestehen (alle Substanzen zur Reaktion vorhanden, Temperatur, Druck).

Der oder die Hohlkörper eines geeigneten Reaktors kann/können beliebig auf die nötigen Dimensionen skaliert werden. Allerdings muss bei der exothermen Oxidation von tertiären Aminen auf die nötige Temperierung, d.h. Wärmeabfuhr geachtet werden. Hierzu ist ein Reaktor, wobei der Hohlkörper von einem Temperierfluid umspült wird, bestens geeignet. Ein derartiger Reaktor ist in WO 2010/055034 beschrieben. Es hat sich gezeigt, dass im Fall der erfindungsgemäßen Oxidationsreaktion bestimmte Dimensionen vorteilhaft sind. Insbesondere beträgt vorzugsweise der Innendurchmesser eines Hohlkörpers des Reaktors 0,25 mm bis 10 mm, vorzugsweise 0,5 mm bis 6 mm, insbesondere bevorzugt 0,8 mm bis 4 mm. Die Länge eines Hohlkörpers des Reaktors ist vorzugsweise 0,5 m bis 20 m, bevorzugt 1 m bis 10 m, insbesondere 2 m bis 5 m. Auch andere Geometrien sind möglich. Bei dickeren Hohlkörpern kann eine größere Oberfläche zum erhöhten Temperaturaustausch notwendig sein. Der Temperaturaustausch des innengeführten Reaktionsfluids kann auch dadurch verbessert werden, dass die Edukte in niedrigerer Konzentration zugeführt werden, oder durch höhere Strömungsgeschwindigkeiten des Rektionsfluids, oder über das außen geführte Temperierfluid durch Erhöhen der Strömungsgeschwindigkeit, oder die Wahl eines Temperierfluids mit höherer Wärmekapazität.

Zur Oxidation des tertiären Amins kann ein Oxidationsmittel zugeführt werden. Eine Gruppe von Oxidationsmitteln sind beispielsweise Peroxide, wobei Wasserstoffperoxid am meisten bevorzugt ist. Andere Peroxide sind z.B. Percarbonsäuren. Andere Oxidationsmittel wie z.B. Sauerstoff oder Ozon können ebenso zum Einsatz kommen. Das tertiäre Amin wird vorzugsweise mit einem Molverhältnis zum Oxidationsmittel von 1:0,9 bis 1:1,3, vorzugsweise von 1:1 bis 1:1,1, eingesetzt.

In besonders bevorzugten Ausführungsformen ist das Aminoxid N-Methyl-Morpholin-N-oxid, welches durch Oxidation von N-Methylmorpholin erhalten werden kann. Letzteres ist somit ein bevorzugtes tertiäres Amin.

Erfindungsgemäß hat sich gezeigt, dass die Oxidation des Reaktionsfluids in einem Reaktor wie einem milli- oder mikrostrukturierten Apparat bei einem Oberfläche zu Volumenverhältnis von 0,5 $m^2/m^3$ oder mehr - zumindest auf einer Länge des Hohlkörpers, in der das tertiäre Amin zu 50% (mol.-%) oxidiert wird (erster Bereich, wie oben erklärt), gut durchgeführt werden kann. Die Oberfläche kann durch die Innenfläche des Hohlkörpers bestimmt werden. Das Oberfläche zu Volumenverhältnis wird mathematisch berechnet durch Division der Oberfläche ($m^2$) durch das Volumen ($m^3$), wie dies aus der Angabe "$m^2/m^3$" ersichtlich ist. Hiermit wird die Dimension im Innenraum des Hohlkörpers (Reaktionsrohres) beschrieben. Weiterleitende Hintergrundliteratur zu dieser Größe findet sich in Némethné et al. (Materials Science and Engineering 39(2), 2014: 89-101). Insbesondere bevorzugt sind höhere Oberfläche zu Volumenverhältnisse, z.B. von 1 $m^2/m^3$ oder mehr, 1,5 $m^2/m^3$ oder mehr, oder 2 $m^2/m^3$ oder mehr. Bei diesen Dimensionen können sehr effizient, die Prozessparameter wie Fließgeschwindigkeit und Temperatur bei der Oxidation von tertiären Aminen in einem kontinuierlichen Prozess gesteuert werden. Durch dieses im Vergleich zu Batch-Verfahren hohe Oberfläche zu Volumenverhältnis können große volumensbezogene Wärmeströme (Kühlung und Erwärmung der Edukte und des Reaktionsfluid) und intensives Mischen ermöglicht werden. Diese intensive Mischung wiederum stellt sicher, dass die Reaktion sehr spezifisch und vollständig abläuft. Der Bereich "auf einer Länge des Hohlkörpers, in der das Amin zu 50% (mol.-%) oxidiert wird" betrifft im erfindungsgemäßen Verfahren den Bereich des Hohlkörpers, in dem in der zumindest die Hälfte des Amins oxidiert wird. Dies ist leicht durch einen Fachmann durch Wahl der Strömungsgeschwindigkeit und Konzentration der eingesetzten reagierenden Substanzen (tert. Amin, ggf. Oxidationsmittel wie Peroxid) steuerbar.

Ein weiterer Parameter zur Charakterisierung des erfinderischen Verfahrens ist die spezifische Oberflächenbelastung, welche im Bereich von 1 $l/m^2h$ bis 40 $l/m^2h$ sein sollte, vorzugsweise von 5 $l/m^2h$ bis 30 $l/m^2h$ oder von 10 $l/m^2h$ bis 20 $l/m^2h$. Die spezifische Volumenbelastung weist einen alternativen oder weiteren Kennwert des erfindungsgemäßen Verfahrens auf, welcher von 1.000 $l/m^3h$ bis 30.000 $l/m^3h$ sein sollte, vorzugsweise von 1.500 $l/m^3h$ bis 25.000 $l/m^3h$, insbesondere bevorzugt von 2.000 $l/m^3h$ bis 20.000 $l/m^3h$. Diese spezifischen Belastungen, je nach Volumen im Hohlkörper oder inneren Oberfläche des Hohlkörpers, zeigen die Dimensionierung des Reaktors bzw. des darin transportierten Fluids zur Reaktoroberfläche, mit der die Reaktionsbedingungen im Reaktionsfluid kontrolliert werden können. Die "spezifische Oberflächenbelastung" ($l/m^2h$) und "spezifische Volumenbelastung" ($l/m^3h$) sind bekannte Begriffe, die zusätzlich durch die Angabe der Einheiten klar sind. Der Begriff der Oberflächenbelastung (auch "Volumen-Oberflächenbelastung" oder in engl. volume surface loading) wird der Publikation Hoffmann et al. (Combination of Anaerobic Treatment and Nutrient Removal of Wastewater in Brazil, 2010) und in Hessel et al. (Micro Process Engineering, A comprehensive Handbook, Wiley-VCH, 2009), in letzterer als Masse-zu-Oberflächenbelastung ($kg/m^2s$), verwendet. Weiterlei-tende Hintergrundliteratur zur Größe der Volumenbelastung findet sich in Windsperger et al. (in Margesin et al. (Herausgeber), Praxis der biotechnologischen Abluftreinigung, Springer 1996, S. 29). Diese Größen geben einen Durchsatz des Reaktionsfluids (hier in $l = 10^{-3} m^3$) durch den Hohlkörper pro Volumen (in $m^3$) bzw. pro Oberfläche (in $m^2$) des Hohlkörpers und pro Zeit (hier in h) an.

Diese Parameter (spezifische Oberflächenbelastung und spezifische Volumenbelastung und auch Oberfläche-zu-VolumenVerhältnis) sind so gewählt, dass selbst bei laminarer Strömung eine effiziente Umsetzung und Reaktion im Hohlkörper erfindungsgemäß erreicht wird - insbesondere in einem milli- oder mikrostrukturierten Rohrreaktor zum kontinuierlichen Betrieb. Selbstverständlich können diese Parameter kombiniert werden, z.B. alle 3 oder das Oberfläche zu Volumenverhältnis (als Dimensionsgröße des Hohlkörpers) mit der spezifischen Oberflächenbelastung oder der spezifischen Volumenbelastung, vorzugsweise mit der spezifischen Oberflächenbelastung.

Eine effiziente Reaktion in einem Rohrreaktor mit kleinem Durchmesser bzw. den angegebenen Oberfläche zu VolumenVerhältnissen ist nicht leicht optimierbar. Einerseits haben kleine Rohrdurchmesser (Kapillaren) Vorteile einer effizient ablaufenden Reaktion (Ausbeute und Geschwindigkeit aufgrund hoher Oberfläche). Andererseits begrenzen die kleinen Rohr- oder Kapillardurchmesser den Fluiddurchsatz im Zusammenhang mit den in den Kapillaren auftretenden, fluidgenerierten Druckverlusten. In Summe konnten überraschenderweise unter Abwägung dieser Vor- und Nachteile bei den erfindungsgemäßen Parameter dennoch eine überraschend gut ablaufende Reaktion festgestellt werden.

Das Reaktionsfluid wird üblicherweise bei laminarer Strömung im Reaktor umgesetzt. In Hohlkörper wird das Reaktionsfluid mit laminarer Strömung geführt. Eine laminare Strömung ist von einer turbulenten Strömung zu unterscheiden. Die turbulente Strömung ist die Bewegung von Fluiden, bei der Verwirbelungen über einen weiten Bereich von Größenskalen auftreten. Hierbei treten hohe Reibungs- und Druckverluste auf, welche zu höherer Wärmeentwicklung führen, insbesondere im erfindungsgemäßen milli- oder mikrostrukturierten Reaktor. Im Gegensatz dazu tritt bei der laminaren keine Verwirbelung auf. Turbulente und laminare Strömung sind durch Viskosität des bewegten Fluids, der Rohrdimension (charakteristische Länge, Querschnitt oder Durchmesser), und der gewählten Strömungsgeschwindigkeit steuerbar. Ein Maß hiervor ist die Reynolds-Zahl, welche - wie in der Literatur bekannt - definiert ist als $Re = \rho vd/\eta$ oder $Re = vd/v$, wobei $\rho$ die Dichte des Fluids ist, $v$ die Strömungsgeschwindigkeit des Fluids ist, $d$ die charakteristische Länge des Hohlkörpers (Durchmesser, Breite oder Höhe) ist ist, $\eta$ die dynamische Viskosität und $v$ die kinematische Viskosität ist. Eine Reynolds-Zahl von 2300-2500 ist üblicherweise der Umschlagsbereich zwischen laminarer und turbulenter Strömung, wobei laminare Strömungen bei kleinerer Reynolds-Zahl zu finden ist. Daher wird erfindungsgemäß vorzugsweise das Reaktionsfluid in Strömung bei einer Reynolds-Zahl von 2300 oder kleiner durch den Hohlkörper geführt, vorzugsweise bei einer Reynolds-Zahl von 2000 oder kleiner, bevorzugt 1000 oder kleiner, insbesondere bevorzugt bei einer Reynolds-Zahl von 1500 bis 1, oder 1 bis 100, oder 1200 bis 10. In den nachfolgenden Beispielen liegen sogar Reynolds-Zahlen im Bereich von 2-30 oder 3-11 vor, welche ebenfalls bevorzugt sind. Weitere mögliche Reynoldszahlen sind 800 oder kleiner, 500 oder kleiner, 300 oder kleiner, z.B. in Kombination mit den genannten unteren Werten.

Beispielswerte zur Berechnung der Reynolds-Zahl sind bekannte Stoffgrößen. Z.B. kann NMM von Alfa Aesar (Katalog Nr. A12158) verwendet werden, welches gemäß Produktdatenblatt eine dynamische Viskosität $\eta$ bei 20°C mit 0,91 mPas hat. Die Dichte $\rho$ ist bei 20°C mit 0,92 g/cm$^3$ > 920 kg/m$^3$. Bei einem Kapillardurchmesser im Hohlreaktor von z.B. 1 mm und einer Fluidgeschwindigkeit von 0,21 bis 0,62 m/min ergeben sich eine Reynolds-Zahl von 3 bis 10. All diese Parameter sind unabhängig vom Hersteller und den beispielhaften Substanzen bevorzugte Größen der erfindungsgemäßen Fluide, des Hohlkörpers und Prozessparameter.

Vorzugsweise hat das Reaktionsfluid eine dynamische Viskosität von 0,1 mPas bis 10 mPas bei 20°C oder bei Reaktionsbedingungen. Vorzugsweise hat das Reaktionsfluid eine Dichte von 0,1 g/cm$^3$ bis 2 g/cm$^3$ bei 20°C oder bei Reaktionstemperatur.

Nachdem die Oxidation von tertiärem Amin zu Aminoxid einer großen Wärmetönung unterliegt, im Fall von NMM zu NMMO mit einem adiabaten Temperaturanstieg bis 400 K und einer spez. Reaktionsenthalpie von bis zu -1600 kJ/kg, kann dies in besonders guter Art und Weise mit dem erfindungsgemäß dargestellten Verfahren ermöglicht werden. Die Handhabung solch extrem exothermer Reaktionen, wie die Oxidierung von NMM zu NMMNO erfordert extrem hohe volumen- und durchsatzspezifische Wärmeströme. Dies bedeutet im erfindungsgemäßen Verfahren, dass punktuelle Überhitzungen vermieden werden (Hot Spots), welche sehr leicht und unkontrolliert in Batchreaktoren auftreten können. Die intensive Energieabfuhr des erfindungsgemäßen Verfahrens ermöglicht es, dass die Geschwindigkeit der Oxidation von tertiärem Amin in Aminoxid unter entsprechender Temperatur- und Druckanpassung angehoben werden kann, ohne dass im Rohrreaktor nennenswerte, räumlich vorherrschende Temperaturunterschiede vorkommen, die extrem betrachtet das "thermische Durchgehen" der Reaktion bedeuten könnten und sich zusätzlich negativ auf die Selektivität der Reaktion auswirken würden. Mit dem erfindungsgemäß dargestellten Verfahren wurde gezeigt, dass die Herstellung von Aminoxid unter kontrollierteren Prozesstemperaturen und höheren Drücken gegenüber dem Stand der Technik erfolgen kann.

Im zweiten Bereich, also einen Bereich nachdem bereits 50% (mol.%) des tertiären Amins oxidiert wurden, können niedrigere Oberfläche zu Volumenverhältnisse gewählt werden, wobei auch zum ersten Bereich überlappende Werte möglich sind. Vorzugsweise ist im zweiten Bereich das Oberfläche zu Volumenverhältnis 5 m$^2$/m$^3$ oder kleiner, besonders bevorzugt 4 m$^2$/m$^3$ oder kleiner, 3 m$^2$/m$^3$ oder kleiner oder 2 m$^2$/m$^3$ oder kleiner.

Selbstverständlich sind auch andere Bereichseinteilungen mit unterschiedlichen Temperaturbedingungen, insbesondere eine andere Temperierung, also ein anderer Wärme(ab)transport vom reaktionsfluid, möglich. Solche Bereichseinteilungen beziehen sich auf den Fortschritt der Oxidation des tertiären Amins, also z.B. eine Bereichseinteilung in Teile des Reaktionsfluids bis zu Reaktionsfortschrittsgrenzen an denen 25%, 33% oder 50%, des tertiären Amins oxidiert wurden.

Anhand der Flussrate (oder Strömungsgeschwindigkeit) des Reaktionsfluides (mit dem Ausgangsstoff) können bei einem entsprechend dimensionierten Hohlkörper diverse Parameter eingestellt werden, beispielsweise die Verweilzeit des tertiären Amins im Reaktor. Die Verweilzeit ist vorzugsweise von 0,4 Minuten bis 14 Minuten, bevorzugt 0,6 Minuten bis 8 Minuten. Mittels der Verweilzeit kann auch der Reaktionsfortschritt in einzelnen Bereichen gesteuert werden. Der Zusammenhang der Verweilzeit und der Oxidation verhält sich annähernd wie eine chemische Reaktion der 2. Ordnung. Der Umsatz und die Verweilzeit einer Reaktion 2. Ordnung hängen über folgende Formel zusammen:

$$X_A = \frac{\tau c_{A,0} k}{1 + \tau c_{A,0} k}$$

k......Geschwindigkeitskonstante der chemischen Reaktion in $\text{lmol}^{-1}\text{s}^{-1}$
$c_{A,0}$...Ausgangskonzentration vom Edukt A (tertiäres Amin) in $\text{moll}^{-1}$
τ......Verweilzeit in s
$X_A$......Umsatz des Eduktes A (tertiäres Amin) als Anteil

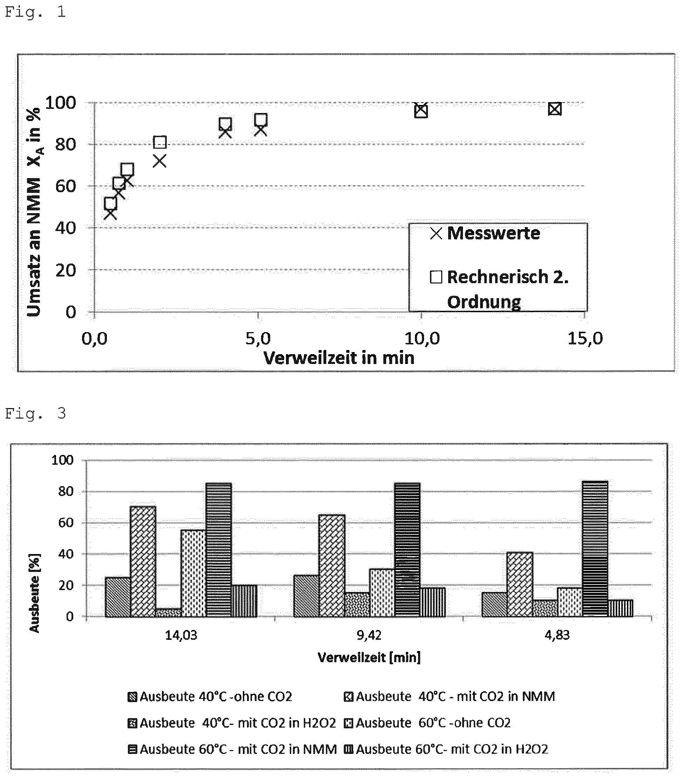

Derartige Verweilzeiten zu Umsätzen sind für den Fall von NMM in Fig. 1 dargestellt. Wie gesagt, es können Verweilzeiten über die Strömungsgeschwindigkeit eingestellt werden. Die Strömungsgeschwindigkeit beeinflusst auch die Temperierung und die Einteilung des Reaktors in entsprechende Bereiche, da mit Fortschreiten der Reaktion tertiäres Amin verbraucht wird, seine Konzentration daher sinkt und folglich auch die Reaktion sich verlangsamt, womit in weiter Folge auch weniger Wärme abgeführt wird. Eine bevorzugte Strömungsgeschwindigkeit des Reaktionsfluids, die für alle Faktoren geeignet ist, ist 0,1 m/min bis 60 m/min, bevorzugt 10 m/min bis 40 m/min, am meisten bevorzugt 20 m/min bis 25 m/min. Weitere bevorzugte Strömungsgeschwindigkeiten des Reaktionsfluids, die ebenso für alle Faktoren geeignet ist, ist 0,1 m/min bis 200 m/min, bevorzugt 10 m/min bis 160 m/min, am meisten bevorzugt 10 m/min bis 120 m/min.

Es ist auch möglich unterschiedliche Strömungsgeschwindigkeiten in unterschiedlichen Bereichen (insbesondere wie oben definiert, je nach Reaktionsfortschritt) auszuwählen. Unterschiedliche Strömungsgeschwindigkeiten ergeben sich bei unterschiedlichen Hohlkörperquerschnitten in den unterschiedlichen Bereichen.

Zusätzlich zur Kontrolle ist somit alternativ oder in Kombination eine unterschiedliche Reaktionsgeschwindigkeit in einzelnen Bereichen steuerbar.

Vorzugsweise ist die spezifische Reaktionswärme zwischen 540 kJ/kg und 1610 kJ/kg, bevorzugt zwischen 1110 kJ/kg und 1420 kJ/kg. Dieser Wert wird insbesondere bevorzugt im ersten Bereich (Reaktion bis 50% des tertiären Amins, wie oben definiert) eingehalten.

Höhere Reaktionsgeschwindigkeiten können bei der Aminoxidherstellung durch Erhöhung der Prozesstemperatur erreicht werden. Allerdings besteht aufgrund der starken Exothermie der chemischen Umwandlung vom tertiären Amin (wie NMM) ins gebildete Aminoxid (wie NMMNO) die Gefahr einer Selbstbeschleunigung, begleitet von einem unkontrollierten Temperatur- und Druckanstieg. Nebenbei wird die Produktqualität in einen unkontrollierten höheren Temperaturbereich negativ beeinflusst. Die Selektivität der Umsetzungsreaktion sinkt und unerwünschte Nebenprodukte können gebildet werden. Solche Nebenprodukte können ausgehend vom tertiären N-Methyl-Morpholin (NMM) beispielsweise Morpholin (M) und Nitrosomorpholin (NMOR) sein. Aus diesen Gründen ist die Reaktionstemperatur im Hohlkörper bzw. des Reaktionsfluids vorzugsweise 20°C bis 70°C, bevorzugt 50°C bis 65°C, insbesondere bevorzugt 40°C bis 65°C, am meisten bevorzugt von 55°C bis 65°C, z.B. 60°C bis 65°C. Diese Temperatur kann durch eine entsprechende temperierte Ummantelung der Hohlkörper des Reaktors eingestellt werden.

Ein bevorzugter Druck im Hohlkörper ist 1 bar bis 200 bar, bevorzugt 5 bar bis 100 bar, am meisten bevorzugt 50 bar bis 100 bar. Vorzugsweise Drücke oder Druckbereiche für die erfindungsgemäße Reaktion sind (alle in bar) 1, 10, 20, 30, 40, 50, 60, 70, 80, 90, 100, 110, 120, 140, 160, 180, 200 oder Werte zwischen diesen Drücken. Bei diesen Drücken findet eine rasche Reaktion statt. Des Weiteren lässt sich bei hohen Drücken $CO_2$ besser lösen und somit mit dem Produktfluid vermischen.

Die Hohlkörper können aus diversen Materialien bestehen, z.B. Metallen, Keramik oder Kunststoffen. Die Innenwand des Hohlkörpers kann einen Einfluss auf die Oxidationsreaktion haben. Vorzugsweise enthält die Innenwand eines Hohlkörpers des Reaktors ein oder mehrere Metalle, vorzugsweise ausgewählt aus Fe, Cr, Ni, Mo, Mn, Nb, Au, Pt, Ti oder Mischungen hiervon. Besonders geeignet sind Edelstähle der Klassen 1.43, 1.44 und 1.45, da diese eine sehr hohe Wärmeleitfähigkeit bei gleichzeitig guter Korrosionsbeständigkeit gegenüber stark oxidativen Medien (wie z.B. $H_2O_2$) besitzen. Vorzugsweise enthält eine Innenwand eines Hohlkörpers Austenit-Stahl. Zusätzlich sollte das Hohlkörpermaterial druckbeständig sein - je nach gewünschtem Reaktionsdruck. Diese Kombination von hoher Wärmeleitfähigkeit, hoher Korrosionsbeständigkeit und hoher Druckbeständigkeit verlangt meist Metall oder Keramikmaterialien. Kunststoffe können für Einbauten (z.B. statischen Mischern) oder zusätzliche Beschichtungen verwendet werden. Als Einlagen bzw. Einbauten, welche vor allem zum Zwecke der besseren Durchmischung der Reaktionslösung eingesetzt werden, benötigt es lediglich eine hohe Korrosionsbeständigkeit, wobei hier Materialien, wie die oben genannten, insbesondere auch Eisen, Titan, Gold, Platin, Keramik, Glas, PTFE, PEEK etc. Einsatz finden.

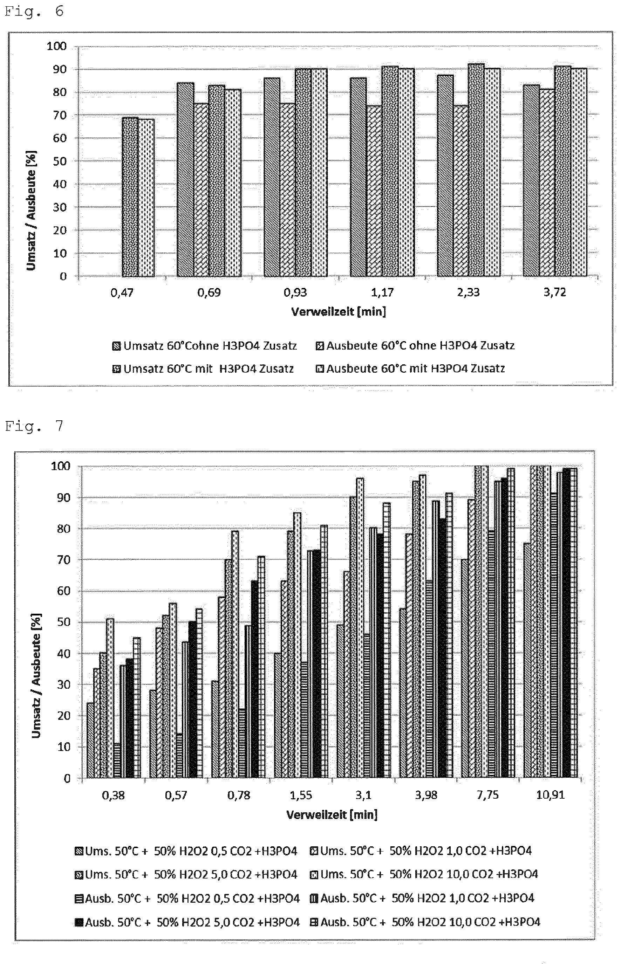

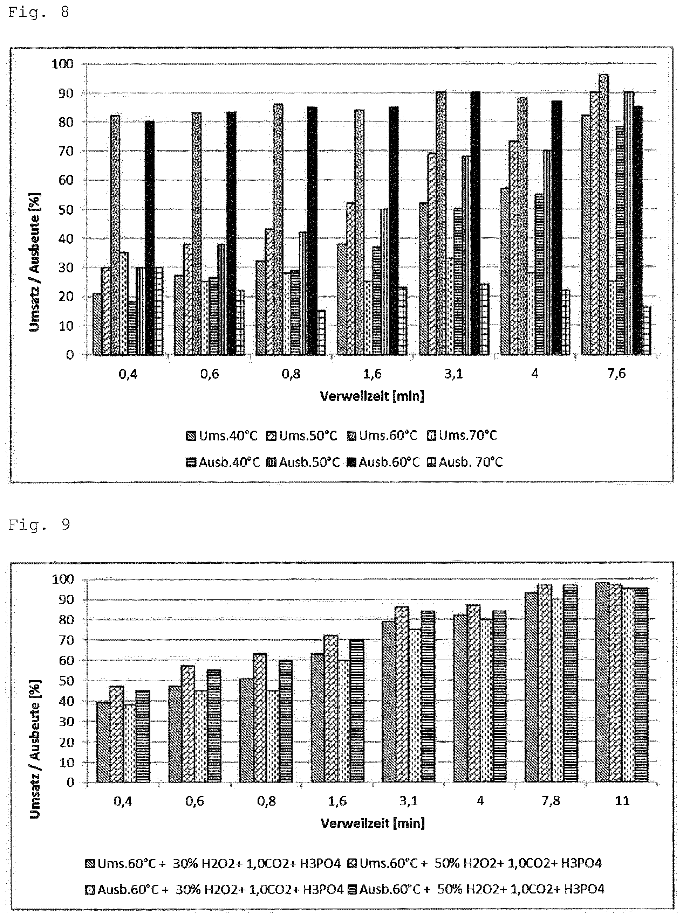

Die Hohlkörper können jede beliebige Form einnehmen, z.B. der Innenquerschnitt kann rund, kreisförmig, quadratisch, halbkreisförmig, hexaedrisch, oktaedrisch usw. sein. Vorzugsweise sind die Hohlkörper Rohre, auch vorzugsweise mit rundem, kreisförmigem, quadratischen, halbkreisförmigen, hexaedrischen, oktaedrischen usw. Innenquerschnitt. Der erfindungsgemäße Reaktor wird hierin auch Rohrreaktor bezeichnet. Zusätzlich sind Einbauten, möglich, welche in beliebig gewählten Abständen oder auch vereinzelt vorgesehen werden. Derartige Einbauten sind beispielsweise Verengungen oder Quetschungen zur Erhöhung der Turbulenz der Strömung, insbesondere zum besseren Vermischen des Fluids im Rohr. Ebenso kann ein oder mehrere statische Mischer (z.B. Mischplättchen) oder Lochplatten vorgesehen werden. Mischzeiten von 1ms bis 1s sind bevorzugt. Geeignete Lochplatten zum Vermischen werden beispielsweise in Fig. 14 der WO 2010/055034 (unter Bezugnahme hierin aufgenommen) gezeigt. Geeignete Quetschungen sind in Fig. 7 und 8 der WO 2010/055034 gezeigt.

Das tertiäre Amin kann in einer Konzentration von 40% bis 100%, vorzugsweise von 50% bis 99,5%, insbesondere 60% bis 99%, speziell bevorzugt 70% bis 99%, oder auch 60% bis 98% oder 70% bis 97%, in den Reaktor zugeführt werden (alle % in vol.-%). Diese Konzentrationen können auch zu Beginn der Reaktion vorliegen, ggf. verdünnt je nach Volumen eines zugesetzten Fluids mit Oxidationsmittel oder anderer Zusätze.

Vorzugsweise wird ein Oxidationsmittel, insbesondere Wasserstoffperoxid, in einer Konzentration von 5% bis 80%, vorzugsweise von 10% bis 70%, insbesondere 20% bis 60%, speziell bevorzugt 30% bis 55%, in den Hohlkörper zugeführt (alle % in Gew.-%). Die Menge sollte mit dem tertiären Amin abgestimmt werden, sodass die obigen Verhältnisse, insbesondere 1:1 moläquivalent tertiäres Amin zu Oxidationsmittel, insbesondere Peroxid, erfüllt sind.

Das Reaktionsfluid kann bei einer Bodensteinzahl von größer als 10, bevorzugt von 20 bis 200, gemäß der Formel

$$Bo = u * L/D_{ax},$$

worin Bo die Bodensteinzahl ist, u die Strömungsgeschwindigkeit des Reaktionsfluids, L die Länge des Hohlkörpers des Reaktors und $D_{ax}$ der axiale Dispersionsstrom des Reaktionsfluids ist, durch den Reaktor befördert werden. Hierzu kann eine geeignete Strömungsgeschwindigkeit bei gegebener Reaktor/Hohlkörpergeometrie ausgewählt werden. Die Bodensteinzahl beschreibt den Grad der Rückvermischung im Reaktor. Sie ist das Verhältnis zwischen dem Konvektionsstrom (u*L) und dem axialen Dispersionsstrom. Im Bereich der gewählten Bodensteinzahlen ergeben sich für die Einbringung des Amins und des Oxidationsmittels zur Bildung des Reaktionsfluides und in weiterer Folge der Oxidation geeignete Mischzustände.

Vorzugsweise wird $CO_2$ in einer Menge von 0,5 Gew.-% bis 20 Gew.-%, bezogen auf das tertiäre Amin, in das Reaktionsfluid eingebracht. $CO_2$ kann zusammen mit dem Amin oder dem Oxidationsmittel oder separat, vorzugsweise nach vorheriger Sättigung in Lösung oder bei mindestens 90 % der Sättigungskonzentration in der jeweiligen Lösung, eingebracht werden oder direkt in den Mikroreaktor eingespeist werden. Vorzugsweise ist die Lösung von $CO_2$ basisch, z.B. mit pH 8-11 oder 9-10, um die Löslichkeit von $CO_2$ zu erhöhen. Vorzugswese wird $CO_2$ im tertiären Amin, ggf. mit Wasser gelöst, oder direkt in das Reaktionsfluid eingebracht, z.B. fluid unter Druck oder als Lösung in Wasser. $CO_2$ kann die Oxidation von tertiären Aminen zu Aminoxid erheblich beschleunigen, im Fall von NMM zu NMMO um das 460-fache. Mechanistisch kommt es zu einer Reaktion von Peroxid ($RHO_2$, insbesondere $H_2O_2$) mit $CO_2$ und das gebildete Peroxymonocarbonat $HCO_4^-$ oder auch $RCO_4^-$ wirkt beschleunigend auf die Reaktion von NMM zu NMMO bzw. ist selbst das aktivere Oxidationsmittel als die eingesetzte Oxidform (wie $H_2O_2$). Da somit $CO_2$ selbst als Reaktionspartner eingreifen kann, kann eine hohe Konzentration die Reaktion positiv beeinflussen. Bei Versuchen mit dem erfindungsgemäßen Reaktor wurde festgestellt, dass besonders hohe und effektive $CO_2$ Mengen (z.B. wenn vollständig oder nahezu vollständig gelöst) eingesetzt werden können. Vorzugsweise wird $CO_2$ unter Druck und gelöst dem Reaktionsfluid zugeführt. Vorzugsweise sind die oben genannten $CO_2$-Mengen gelöstes $CO_2$. Im erfindungsgemäßen kontinuierlichen System können besonders hohe $CO_2$ Mengen erreicht werden und diese haben positive Auswirkungen auf die Reaktion. Beispielsweise kann die $CO_2$ Menge mindestens 0,5%, mindestens 1%, mindestens 2%, mindestens 4%, mindestens 6%, mindestens 8%, mindestens 10%, mindestens 15%, alle Gew.-%, bezogen auf das tertiäre Amin oder eine andere in das Reaktionsfluid eingebrachte Lösung von $CO_2$, betragen. Ebenfalls sind diese Mengen bezogen auf das gesamte Reaktionsfluid möglich.

Zusätzlich kann ein Metallkomplexbildner, vorzugsweise ein Phosphat, dem Reaktionsfluid zugefügt werden, vorzugsweise in einer Menge von 0,01% bis 3%, vorzugsweise 0,03% bis 2%, speziell bevorzugt 0,05% bis 1,5% (alle Gew.-%). Unerwünschte Metalle im Fluid können das Oxidationsmittel zersetzen und daher die Reaktivität gegenüber dem tertiären Amin senken. Der Metallkomplexbildner soll daher diese Wirkung des Metalls verhindern oder reduzieren. Jeder beliebige Komplexbildner, z.B. Chelatbildner, ist möglich (siehe z.B. einleitend erwähnt). Überraschenderweise wurde festgestellt, dass Phosphat ebenfalls geeignet ist, insbesondere bei eisenhaltigen Reaktormaterialien, welche Eisen abgeben können bzw. deren Eisenmantel selbst an der Oberfläche zum Reaktionsfluid störend einwirken kann. Dies konnte mittels Metallkomplexbildner reduziert werden, sodass etwa 10% höhere Ausbeuten erzielt werden konnten.

Vorzugsweise wird das tertiäre Aminoxid vorgewärmt bevor es oxidiert wird. Das Vorwärmen ist insbesondere auf die im Reaktionsfluid gewünschte Temperatur, z.B. ausgewählt aus den oben genannten Temperaturen. Vorwärmen kann in einem Reaktor oder Hohlkörper wie oben beschrieben vorgenommen werden und kann bei entsprechend hohen Oberflächen zu Volumenverhältnissen relativ rasch erfolgen (z.B. Verhältnisse wie während der oben dargestellten Reaktion). Vorwärmen kann beispielsweise in einem Zeitraum von 5 sec bis 5 min erfolgen, vorzugsweise etwa 1 min. Etwa bedeutet +/- 20%.

Ebenso kann das Oxidationsmittel vorgewärmt werden. Hierzu können ebenso die für das tertiäre Amin beschriebenen Maßnahmen und Parameter gewählt werden, z.B. Vorwärmen in einem Zeitraum von 5 sec bis 5 min, vorzugsweise etwa 1 min.

Nach erwünschtem Reaktionsfortschritt, z.B. wenn das gesamte tertiäre Amin oder beinahe das gesamte tertiäre Amin (z.B. 99,5% oder mehr) oxidiert wurde, kann verbleibendes Oxidationsmittel abgebaut oder entfernt werden. Im Fall von Peroxiden, insbesondere Wasserstoffperoxid, eignet sich der Einsatz von Katalase oder die Behandlung mit einem anderen Katalysator wie $MnO_2$ oder Platin (z.B. platinierter Nickel). Der Katalysator kann in Form von Bündeln oder Partikeln inhärent mit hoher Oberfläche (z.B. Katapak®) oder als Katalysatorblech eingesetzt werden.

Anstatt sauberen tertiären Aminen können auch aufkonzentrierte Brüden, welche bei der Herstellung von Cellulosefasern anfallen, verwendet werden. Die Aufkonzentrierung wurde wie in EP 0 448 924 B1 beschrieben durch Umkehrosmose durchgeführt. Morpholin wurde, wie im EP 0 448 924 B1 beschrieben, in einem abschließenden Schritt abdestilliert und kann nach einer Methylierung, wie beispielsweise im Patent DE 3504899 A1 beschrieben, ebenfalls oxidiert werden.

Das erfindungsgemäß produzierte Aminoxid, vorzugsweise NMMO, kann direkt eingesetzt werden. Das erfindungsgemäße kontinuierliche Verfahren eignet sich hier besonders das laufend produzierte Aminoxid in eine ebenfalls kontinuierlich betriebene Folgeanlage einzusetzen. Ein solcher gekoppelter Folgeprozess ist beispielsweise das Auflösen von Zellulose in NMMO oder anderen Aminoxiden. Wie einleitend erwähnt kann eine Zelluloselösung in Aminoxid für ein Spinnverfahren (Lyocellverfahren) eingesetzt werden. Hierbei können aus der Zelluloselösung Filamente, Fasern oder Folien geformt und hergestellt werden. Auch dieses ist mit dem erfindungsgemäßen Verfahren in-line oder off-line kombinierbar, analog wie der Auflösungsvorgang.

Gegebenenfalls kann das erfindungsgemäß produzierte Amninoxid aufkonzentriert oder verdünnt werden, vorzugsweise nach einem Abbau oder der Entfernung des Oxidationsmittels. Vorzugsweise wird die Konzentration des erhaltenen Aminoxid auf 20%-60%, insbesondere bevorzugt auf 30%-50% eingestellt (alle Gew.-%). Derartige Zusammensetzungen eignen sich gut zur Lagerung oder zum Transport (inklusive zum Transport durch Rohrleitungen in einer gekoppelten Anlage). Zum Auflösen von Zellulose werden vorzugsweise höhere Konzentrationen eingestellt. So kann das produzierte Aminoxid auf eine Konzentration von 50% bis 95%, vorzugsweise 60% bis 90% oder 70% bis 80% eingestellt werden (alle Gew.-%). Als Verdünnungsmittel kann Wasser oder jede andere mischbare Flüssigkeit eingesetzt werden.

Beim Auflösen und/oder zum Spinnen von Zellulose im produzierten Aminoxid wird vorzugsweise eine Lösung mit 4% bis 30%, vorzugsweise 8% bis 25% oder 10% bis 20%, Zellulose erzeugt (alle Gew.-%). Eine geeignete Spinnlösung hat zum Beispiel die Zusammensetzung Zellulose: 12,9%, NMMO 76,3%, Wasser 10,8%, alle % in Gew.-% (siehe WO 2013/030400, durch Bezugnahme hierin aufgenommen). In der WO 2013/030400 wird weiters ein Spinnverfahren beschrieben, welche erfindungsgemäß eingesetzt werden kann. Im Allgemeinen kann die Zelluloselösung mit dem erfindungsgemäß produzierten Aminoxid extrudiert werden, wobei die Extrudate einen Gasspalt passieren und in ein Fällbad eingebracht werden. Das Fällbad kann ein Fällmittel, z.B. Wasser, in einer Konzentration zum Ausfällen der Zellulose aufweisen. Im Gasspalt kann ein Gasstrom in an sich bekannter Weise geführt werden.

Demgemäß umfasst die vorliegende Erfindung auch ein Verfahren, wobei das erfindungsgemäß produzierte Aminoxid auf eine Konzentration zum Auflösen von Zellulose gebracht wird und optional weiters die Zellulose darin aufgelöst wird und optional weiters die aufgelöste Zellulose in Endlosformkörper, z.B. Filamente, Fäden oder Folien, geformt wird, z.B. durch Extrusion, und anschließend die Zellulose der Formkörper gefällt wird.

Reaktorsystem:

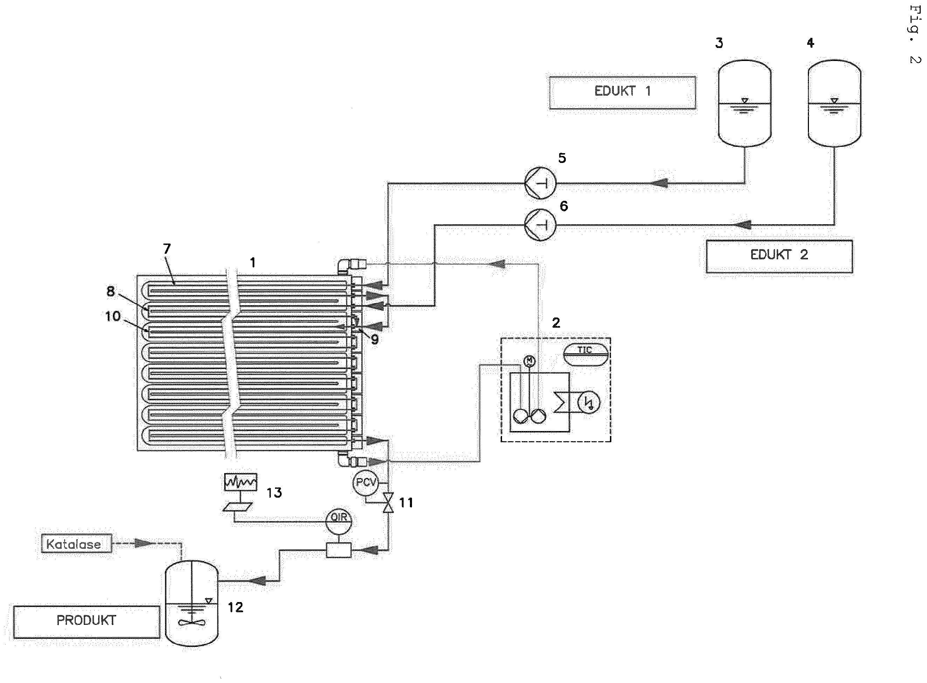

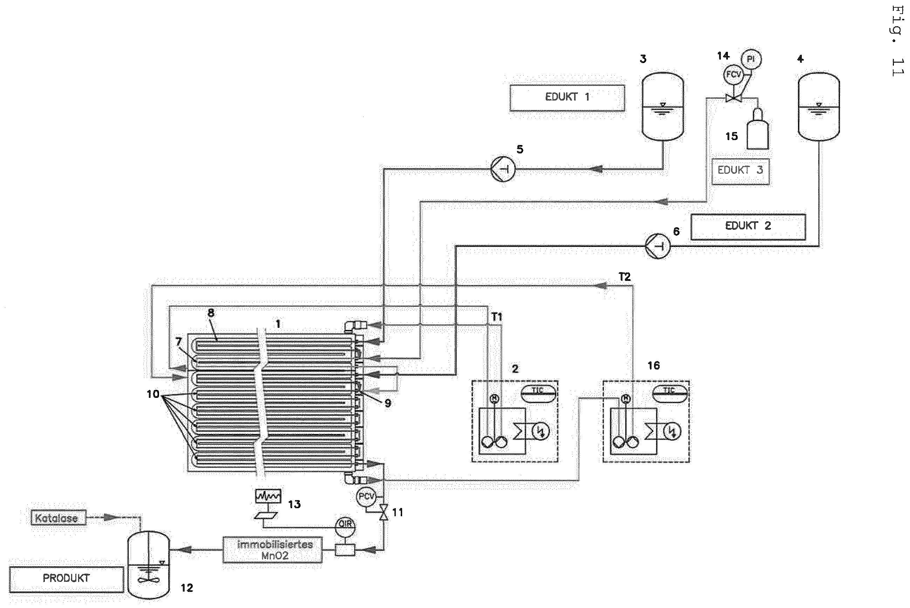

Ein erfindungsgemäßer Reaktor in einem Reaktorsystem ist in Figur 2 und 11 dargestellt. Es wurde ein Mikroreaktor (1) wie in WO 2010/055034 beschrieben eingesetzt. Das Reaktorsystem hat einen Rohrreaktor (1), der zur Führung des Reaktionsfluids verwendet wird, sowie zum Vorheizen der beiden Edukte (tertiäres Amin und Oxidationsmittel). Die Edukte werden aus Vorlagebehältern (3, 4) über Pumpen (5, 6) zum Reaktor (1) geführt. Dort werden in Zonen 7 und 8 die Edukte vorgewärmt. Zum Beheizen wird ein Temperiermedium um die Hohlkörper geführt. Das Temperiermedium wird im Thermostat (2) geheizt oder gekühlt. Die vorgewärmten Edukte werden an der Position 9 zusammengeführt, wodurch das Reaktionsfluid entsteht und durch die Hohlkörper, z.B. Reaktorrohre 10 geführt wird. Druck wird sowohl über die Pumpen (5, 6) als auch über ein Druckhalteventil (11) gesteuert. Das Produkt wird in einem Behälter (12) aufgefangen, welcher über ein Rührwerk verfügt. Hier können optional noch weitere Reaktionen durchgeführt werden, z.B. Durchoxidieren von verbliebenen Amin oder Abbau von Oxidationsmittel. Ein Messgerät mit Durchflusszelle (13) kann optional den Produktabfluss überwachen.

Die vorliegende Erfindung wird weiter durch die folgenden Beispiele erläutert und eingehender beschrieben, ohne auf diese Ausführungsformen der Erfindung beschränkt zu sein. Sämtliche Parameter und Apparatteile dieser Beispiele sind auch isoliert von der restlichen Beispielsbeschreibung mit den oben genannten Hauptaspekten der Erfindung oder den bevorzugten Ausführungsformen kombinierbar.

**Beispiele:**

Chemikalien:

Die zur Durchführung der Experimente eingesetzten Chemikalien wurden in verschiedener Reinheit von unterschiedlichen Lieferanten bezogen. Tabelle 1 zeigt eine Aufstellung der für die Versuchsdurchführung benutzten Chemikalien.

Tabelle 1: Chemikalien

| Bezeichnung | Lieferant | Reinheit [%] | Katalognummer | CAS-Nummer |
|---|---|---|---|---|
| NMM | Alfa-Aesar | $\geq$ 99 | A12158 | 109-02-4 |
| $H_2O_2$ 30%ig | Sigma | - | 216763 | 7722-84-1 |
| $H_2O_2$ 50%ig | Sigma | - | 516813 | 7722-84-1 |
| Katalase (lagern bei -20°C) | Sigma | 2,000 - 5,000 units/mg | C-9322 | 9001-05-2 |
| $CO_2$ N45 | Air Liquide | 99,995% | | |
| Kaliumpermanganat | Sigma | 0,2 M | 35184 | 7722-64-7 |
| Phosphorsäure | Carl Roth | 75%, reinst. | 2614.1 | 7664-38-2 |
| Schwefelsäure Kühlmittel Wasser/ Ethylenglykol 1/1 Vol./Vol. | Bernd Kraft | 2,5 M | 03279.3000 | |

**HPLC-UV**

| Bezeichnung | Lieferant | Reinheit [%] | Katalognummer | CAS-Nummer |
|---|---|---|---|---|
| Morpholin | Sigma | $\geq$ 99 | 252360 | 110-91-8 |
| NMMNO $_x H_2O$ | Alfa-Aesar | 98+ | A15996 | 70187-32-5 |
| NMMNO 50 Gew.% in $H_2O$ | Alfa-Aesar | - | A19802 | 7529-22-8 |

**Reaktionssystem nach Fig. 2**

Lösen von $CO_2$ in N-Methylmorpholin

Zur Erhöhung der Reaktionsgeschwindigkeit wird Edukt 1 mit [F] Gew.-% $CO_2$ gesättigt. Dies passierte z.B. durch das Eintragen von $CO_2$ über ein Gaseinleitungsrohr oder eine gesinterte, poröse Vorrichtung in die NMM-Lösung. NMM wurde auch mit einer $CO_2$-Atmosphäre überlagert um unter Rühren bei 0,1-20 bar Überdruck $CO_2$ in NMM zu lösen. Nach abgeschlossener Dosierung wurde die jeweilige $CO_2$ angereicherte Eduktlösung zur Kontrolle abgewogen und für die Mikroreaktorversuche eingesetzt.

Reaktionsführung im Mikroreaktor

Das von uns verwendete Mikroreaktionssystem basiert auf der WO 2010/055034 und wurde aus dem Werkstoff [A] gemäß Tabelle hergestellt. Die Mikroreaktorversuche der Synthese von N-Methylmorpholin-N-oxid (NMMNO) aus 4-N-Methyl-Morpholin (NMM) und Wasserstoffperoxid ($H_2O_2$) wurden in unterschiedlichen Aufbauten im Mikroreaktor durchgeführt. Die Kenndaten des eingesetzten Reaktors (1) waren wie folgt:

Volumen: variabel, bis zu 26 mL

Kapillardurchmesser: 1 mm, Rohre mit Quetschungen Da die Umsetzung von NMM mit $H_2O_2$ zu NMMNO exotherm (adiabater Temperaturanstieg 335 K) erfolgt, ist bei dieser Reaktion sicherzustellen, dass die Mikroreaktorbedingungen und technische Auslegung so gewählt sind, dass die Bedingungen für den Wärmetransport ausreichend geeignet sind, sodass dem Reaktionssystem einerseits dem Edukt NMM Wärme zugeführt, andererseits aus der Reaktionslösung Wärme abgeführt werden kann. Dies geschieht durch ausreichend dimensionierte Mikroreaktoroberflächen. Dank der großen Austauschfläche zwischen Reaktionsmedium und Temperiermedium kann die Wärme kontrolliert abgeführt werden und es erübrigen sich lange Dosierzeiten, welche im Batchverfahren bei mehreren Stunden liegen. Des Weitern befinden sich im Mikroreaktor kleinere Reaktionsvolumina, wodurch die Sicherheit des Verfahrens erheblich gesteigert wird. Durch die verbesserte Prozesskontrolle können eine verbesserte Qualität, Selektivität und eine höhere Ausbeute verzeichnet werden. Des weiteren ermöglicht der Versuchsaufbau das Arbeiten unter erhöhtem Druck, wodurch sich der Stofftransport/Phasentransfer im Vergleich zum Batchreaktor stark verbessert.

Es wurde ein Heizthermostat (2) ECO SILVER RE415 der Firma Lauda eingesetzt um den Mikroreaktor auf [B] °C zu temperieren. Als Kühl- und Heizmedium wurde ein Gemisch aus Wasser/ Ethylenglycol im Volumensverhältnis 1:1 eingesetzt.

In zwei Vorratsbehältern (3, 4) werden die beiden Edukte vorgelagert.

Tabelle 2: Edukte

|  | Edukt 1 | Edukt 2 |
|---|---|---|
| Volumenstrom | [C] ml/min | [D] ml/min |
| Molares Verhältnis | [E] | |
| Zusammensetzung | 8,5 M N-Metyhlmorpholin vorgesättigt mit [F] Gew.% $CO_2$ | [G] Gew.-%iges Wasserstoffperoxid mit [H] gelöstem $CO_2$ und [I] Gew.% $H_3PO_4$ |

Über zwei computergesteuerte Pumpen (5 und 6) des Typs SyrDos von HiTec Zang werden die Volumenströme der Chemikalien geregelt und nach den Vorwärmrohren (7 und 8) über eine Mischmodul T3 mit eingesetztem Statischen Mischer (Z.B. Mischplättchen) (9), gemäß WO 2010/055034, zusammengeführt. Im Mischmodul können unterschiedliche Mischeinsätze eingesetzt werden. Anschließend wird das Reaktionsgemisch in die Reaktionsrohre (10) geleitet, wo dann die Reaktion stattfindet. Zur Erhöhung und Regulierung des Drucks im Reaktor wurde vor der Durchflusszelle ein Druckhalteventil (11) für den Bereich 0-20 bar eingesetzt und auf [J] bar geregelt. Ein weiteres Druckhalteventil kann nach der Durchflusszelle installiert werden um Gasbildung in der Zelle zu vermeiden. Der Einbau des Druckhalteventils sollte die Zerfalls- und Nebenreaktionen möglichst unterdrücken, da es im Zuge dieser unerwünschten Reaktionen zur Gasentwicklung kommt.

Das gewünschte Produkt wird in einem Gefäß mit Rührwerk (12) aufgefangen. Überschüssiges Wasserstoffperoxid wird durch langsames Zutropfen von 0,5 Gew.-%iger Katalaselösung zersetzt und anschließend einem Peroxidtest unterzogen.

Durchsätze von [K] ml/min mit Umsätzen von [L] % und Ausbeuten von [M] % konnten erreicht werden.

Tabelle 3: Verfahrenstechnische Parameter

| | |
|---|---|
| Fluidgeschwindigkeit | [N] m/min |
| Hydrodynamische Verweilzeit im Reaktionsbereich | [O] min |
| Reaktorspezifische Oberflächenbelastung | [P] l/m$^2$h |
| Reaktorspezifische Volumensbelastung | [Q] l/m$^3$h |
| Oberflächen:Volumen Verhältnis | [R] |

Analytik

Über eine online FT-IR Messung (13) in einer Durchflusszelle mit dem Spektrometer Alpha von BRUKER wird die Qualität des Produkts aufgezeichnet. Der Peak bei 1045-1028 cm$^{-1}$ welcher dem Edukt zugeordnet werden kann wird zur Verfolgung des Umsatzes verwendet. Die Peaks, die dem NMMNO zugeordnet werden können, erscheinen bei den Wellenzahlen 1250-1215 cm$^{-1}$, 995-960 cm$^{-1}$ und 960-920 cm$^{-1}$. Zur Auswertung der Ausbeute wird der Peak bei 1250-1215cm$^{-1}$ verwendet.

Die Reaktionsproben wurden ebenfalls off-line mittels HPLC/UV analysiert. Es werden die Proben mittels Flüssigchromatographie getrennt und über einen UV-Detektor gemessen. Anhand der erhaltenen Chromatogramme werden die Versuche ausgewertet. Zur Analyse wurde ein HPLC 1100 der Firma Agilent Technologies verwendet.

Die erhaltenen Chromatogramme aus der Kalibrierung zeigten NMMNO bei einer Retentionszeit von 1,940 Minuten, sowie NMM mit einer Retentionszeit von 17,341 Minuten und Morpholin mit einer Retentionszeit von 5,461 Minuten an. Das Chromatogramm der Einspritzung eines sythetische hergestellten Referenzgemisches (NMM, NMMNO, M) zeigte, dass die Substanzen sauber voneinander getrennt werden und bestimmt werden können.

Zur Bestimmung von gegebenenfalls gebildetem N-Nitrosomorpholin (NMOR) wurde, auf Grund der zu erreichenden Konzentrationen von < 100 ppb, ein Gaschromatograph mit Massenspektrometrie-Kopplung verwendet, da in diesen Bereichen die Genauigkeit von HPLC/UV Systemen nicht ausreicht. NMOR kann durch Einsatz mittelpolarer Lösungsmittel aus dem Produkt extrahiert werden. Bei der Probenvorbereitung hat sich die Verwendung einer 1:1 Mischung von Dichlormethan (DCM) und Ameisensäureethylester (EF) als zweckmäßig erwiesen.

Das Produkt wurde ebenfalls refraktometrisch bei Brechungsindexen von 1,330 - 1,449 bestimmt. Anhand des Brechungsindex kann die Konzentration des synthetisierten NMMNOs abgelesen werden. Die Konzentration des Produkts wurde auch über die Dichte bestimmt.

$CO_2$-Beimischung

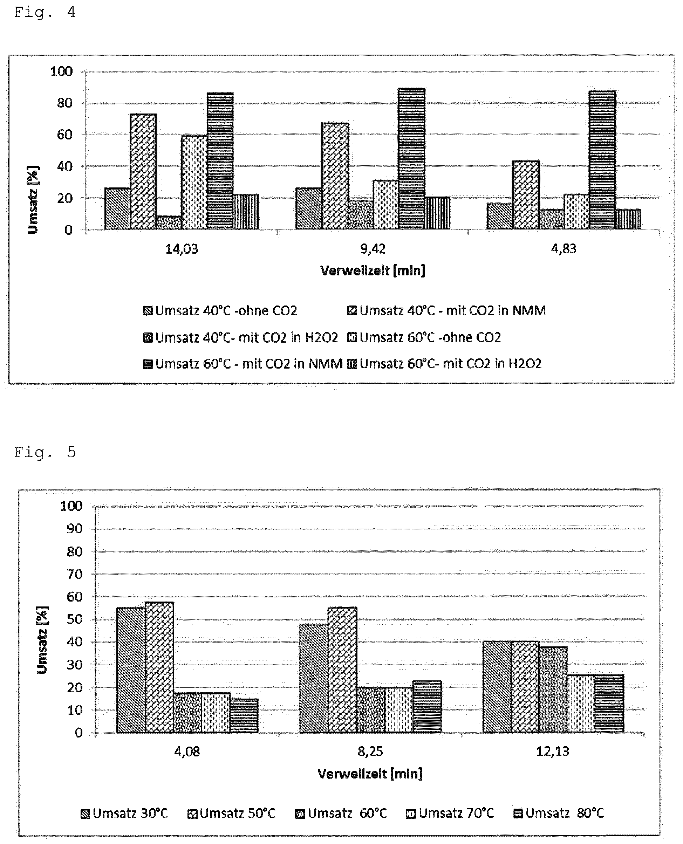

Es wurden Versuche durchgeführt, die zeigen sollten, worin das $CO_2$ für möglichst hohe Umsätze und Ausbeuten gelöst werden soll. Es zeigte sich, dass die Zugabe von $CO_2$ zum NMM sich in allen Fällen positiv auf die Ausbeute auswirkt (+40-70%, Fig. 3 und 4). Der Grund dafür ist in der hohen Löslichkeit von $CO_2$ im N-Methyl-Morpholin zu finden.

Bei kürzeren Verweilzeiten hat eine erhöhte Temperatur größere Auswirkung auf die Ausbeute als bei längeren Verweilzeiten. Die Reaktion läuft bei erhöhten Temperaturen also schneller ab, was die Produktionsrate durch die möglichen kürzeren Verweilzeiten steigert.

Stabilisierung des Wasserstoffperoxids mit Phosphorsäure zur Reaktionsverbesserung

Da sich bei erhöhten Temperaturen immer etwas Wasserstoffperoxid zersetzt und dadurch der Reaktionslösung fehlt wurde versucht, dies durch Phosphorsäure zu unterdrücken. Es konnte festgestellt werden, dass die Umsätze und Ausbeuten durch Zugabe von $H_3PO_4$ um 5-10% erhöht werden (Fig. 6). Dieser Effekt verstärkt sich mit einer Erhöhung der Verweilzeit. Phosphorsäure wirkt als Komplexbildner, welcher katalytisch wirkende Metalllonen, welche das Wasserstoffperoxid zersetzen könnten, komplexiert.

Tabelle 4: Parameter zu Versuchen der $CO_2$-Beimischung

| A | B | C | D | E | F | G | H | I | J | K | L | M | N | O | P | Q | R |
|---|---|---|---|---|---|---|---|---|---|---|---|---|---|---|---|---|---|
| Werkstoff | Temperatur [°C] | Edukt 1 N-Methyl-Morpholin (NMM) [ml/min] | Edukt 2 Wasserstoffperoxid (H2O2) [ml/min] | Molverhältnis Edukt 1 : Edukt 2 [mol/mol] | CO2 in NMM [Masse %] | Konzentration H2O2 [Masse %] | CO2 in H2O2 [Masse %] | H3PO4 in H2O2 [Masse %] | Druck [bar] | Durchsatz [ml/min] | Umsatz [%] | Ausbeute NMMO [%] | Fluidgeschwindigkeit [m/min] | hydrodynamische Verweilzeit im Reaktionsbereich - Reaktorlänge [min] | Reaktorspezifische Oberflächenbelastung [l/m²h] | Reaktorspezifische Volumenbelastung [l/m³h] | Oberfläche: Volumen Verhältnis |
| PTFE | 40 | 0,23 | 0,2 | 1:1 | 0 | 29 | 0 | 0 | 6,89 | 0,43 | 26 | 25 | 0,21 | 14,03 | 1,71 | 4.277,42 | 2,5 |
| | 40 | 0,34 | 0,3 | 1:1 | 0 | 29 | 0 | 0 | 6,89 | 0,64 | 26 | 26 | 0,32 | 9,42 | 2,55 | 6.366,39 | 2,5 |
| | 40 | 0,67 | 0,58 | 1:1 | 0 | 29 | 0 | 0 | 6,89 | 1,25 | 16 | 15 | 0,62 | 4,83 | 4,97 | 12.434,35 | 2,5 |
| | 40 | 0,23 | 0,2 | 1:1 | 0,5 | 29 | 0 | 0 | 6,89 | 0,43 | 73 | 70 | 0,21 | 14,03 | 1,71 | 4.277,42 | 2,5 |
| | 40 | 0,34 | 0,3 | 1:1 | 0,5 | 29 | 0 | 0 | 6,89 | 0,64 | 67 | 65 | 0,32 | 9,42 | 2,55 | 6.366,39 | 2,5 |
| | 40 | 0,67 | 0,58 | 1:1 | 0,5 | 29 | 0 | 0 | 6,89 | 1,25 | 43 | 41 | 0,62 | 4,83 | 4,97 | 12.434,35 | 2,5 |
| | 40 | 0,23 | 0,2 | 1:1 | 0 | 29 | zugef. | 0 | 6,89 | 0,43 | 8 | 5 | 0,21 | 14,03 | 1,71 | 4.277,42 | 2,5 |
| | 40 | 0,34 | 0,3 | 1:1 | 0 | 29 | zugef. | 0 | 6,89 | 0,64 | 18 | 15 | 0,32 | 9,42 | 2,55 | 6.366,39 | 2,5 |
| | 40 | 0,67 | 0,58 | 1:1 | 0 | 29 | zugef. | 0 | 6,89 | 1,25 | 12 | 10 | 0,62 | 4,83 | 4,97 | 12.434,35 | 2,5 |
| | 60 | 0,23 | 0,2 | 1:1 | 0 | 29 | 0 | 0 | 6,89 | 0,43 | 59 | 55 | 0,21 | 14,03 | 1,71 | 4.277,42 | 2,5 |
| | 60 | 0,34 | 0,3 | 1:1 | 0 | 29 | 0 | 0 | 6,89 | 0,64 | 31 | 30 | 0,32 | 9,42 | 2,55 | 6.366,39 | 2,5 |
| | 60 | 0,67 | 0,58 | 1:1 | 0 | 29 | 0 | 0 | 6,89 | 1,25 | 22 | 18 | 0,62 | 4,83 | 4,97 | 12.434,35 | 2,5 |

EP 3 558 901 B1

(fortgesetzt)

| A Werkstoff | B Temperatur [°C] | C Edukt 1 N-Methyl-Morpholin (NMM) [ml/min] | D Edukt 2 Wasserstoff peroxid (H2O2) [ml/min] | E Molverhältnis Edukt 1 : Edukt 2 [mol/mol] | F CO2 in NMM [Masse %] | G Konzentration H2O2 [Masse %] | H CO2 in H2O2 [Masse %] | I H3PO4 in H2O2 [Masse %] | J Druck [bar] | K Durchsatz [ml/min] | L Umsatz [%] | M Ausbeute NMMO [%] | N Fluidgeschwindigkeit [m/min] | O hydrodynamische Verweilzeit im Reaktionsbereich - Reaktorlänge [min] | P Reaktorspezifische Oberflächenbelastung [l/m²h] | Q Reaktorspezifische Volumensbelastung [l/m³h] | R Oberfläche: Volumen Verhältnis |
|---|---|---|---|---|---|---|---|---|---|---|---|---|---|---|---|---|---|
| | 60 | 0,23 | 0,2 | 1:1 | 0,5 | 29 | 0 | 0 | 6,89 | 0,43 | 86 | 85 | 0,21 | 14,03 | 1,71 | 4.277,42 | 2,5 |
| | 60 | 0,34 | 0,3 | 1:1 | 0,5 | 29 | 0 | 0 | 6,89 | 0,64 | 89 | 85 | 0,32 | 9,42 | 2,55 | 6.366,39 | 2,5 |
| | 60 | 0,67 | 0,58 | 1:1 | 0,5 | 29 | 0 | 0 | 6,89 | 1,25 | 87 | 86 | 0,62 | 4,83 | 4,97 | 12.434,35 | 2,5 |
| | 60 | 0,23 | 0,2 | 1:1 | 0 | 29 | zugef. | 0 | 6,89 | 0,43 | 22 | 20 | 0,21 | 14,03 | 1,71 | 4.277,42 | 2,5 |
| | 60 | 0,34 | 0,3 | 1:1 | 0 | 29 | zugef. | 0 | 6,89 | 0,64 | 20 | 18 | 0,32 | 9,42 | 2,55 | 6.366,39 | 2,5 |
| | 60 | 0,67 | 0,58 | 1:1 | 0 | 29 | zugef. | 0 | 6,89 | 1,25 | 12 | 10 | 0,62 | 4,83 | 4,97 | 12.434,35 | 2,5 |

Reaktormaterialien

Die Synthese von NMMNO wurde mit unterschiedlichen Kapillarmaterialien durchgeführt. Auch der hochkorrosionsbeständige Werkstoff Hastelloy® C-22 wurde getestet. Hastelloy Legierungen zählen zur Gruppe der hochkorrosionsbeständigen Nickel-Molybdän Legierungen und sind gekennzeichnet durch hohe Nickel, Molybdän und Chromgehalte. Diese Werkstoffe zeichnen sich durch hohe Beständigkeiten in reduzierenden Medien aus.

Wie in Fig. 5 erkenntlich, wirkt sich eine Erhöhung der Temperatur bis 50°C-60°C gut auf den Umsatz von NMM aus. Diese Versuche wurden ohne $CO_2$ durchgeführt. Bei weiterer Erhöhung zersetzt sich das Wasserstoffperoxid an der Reaktorwand bevor es mit NMM reagieren kann. Das Reaktionsprodukt wird braun-rot und es herrscht massive Gasentwicklung. Hastelloy® C-22 eignet sich ohne Metallmaskierer eher schlecht für diese Reaktion als Reaktormaterial. Da die Reaktion in Edelstählen der Klasse 1.43, 1.44 und 1.45 problemlos abläuft ist ein möglicher Grund dafür der hohe Anteil von Molybdän und/oder Nickel, der die Zersetzung von Wasserstoffperoxid katalysiert. Vorzugsweise wird der Werkstoff Hastelloy® C-22 erfindungsgemäß nicht eingesetzt oder maskiert.

Nichtrostende, korrosionsbeständigen Stähle austenitische Stähle der Stahlgruppennummern 1.40xx bis 1.45xx sind bevorzugte Kapillar-Rohrmaterialen.

Edelmetalle wie Gold/Platin oder die Übergangsmetalle Titan/Tantal und verschiedenste Polymere wie PEEK, PTFE, PFA, PVDF, PMMA, PVC, PET eignen sich ebenfalls hervorragend als Reaktormaterialien, wie Hohlkörper, insbesondere Kapillaren, Rohre und Mischplättchen als Einzelwerkstoff oder in Kombinationen für die Durchführung dieser Reaktion. Verwendete Hohlkörper-, Rohr- und Kapillarmaterialien können auch beschichtet oder emailliert zum Einsatz kommen.

Tabelle 5: Parameter zu Versuchen der Phosphorsäure Beimischung

| A Werkstoff | B Temperatur [°C] | C Edukt 1 N-Methyl-Morpholin (NMM) [ml/min] | D Edukt 2 Wasserstoffperoxid (H2O2) [ml/min] | E Molverhältnis Edukt 1 : Edukt 2 [mol/mol] | F CO2 in NMM [Masse %] | G Konzentration H2O2 [Masse %] | H CO2 in H2O2 [Masse %] | I H3PO4 in H2O2 [Masse %] | J Druck [bar] | K Durchsatz [ml/min] | L Umsatz [%] | N Fluidgeschwindigkeit [m/min] | O hydrodynamische Verweilzeit im Reaktionsbereich-Reaktorlänge [min] | P Reaktorspezifische Oberflächenbelastung [l/m²h] | Q Reaktorspezifische Volumensbelastung [l/m³h] | R Oberfläche: Volumen Verhältnis |
|---|---|---|---|---|---|---|---|---|---|---|---|---|---|---|---|---|
| Hastelloy® C-22 | 30 | 0,18 | 0,16 | 1:1 | 0 | 32 | 0 | 0 | 6,89 | 0,34 | 40 | 0,43 | 12,13 | 1,24 | 4.947,59 | 4 |
| | 30 | 0,27 | 0,23 | 1:1 | 0 | 32 | 0 | 0 | 6,89 | 0,5 | 48 | 0,64 | 8,25 | 1,82 | 7.275,87 | 4 |
| | 30 | 0,54 | 0,47 | 1:1 | 0 | 32 | 0 | 0 | 6,89 | 1,01 | 55 | 1,29 | 4,08 | 3,67 | 14.697,26 | 4 |
| | 50 | 0,18 | 0,16 | 1:1 | 0 | 32 | 0 | 0 | 6,89 | 0,34 | 40 | 0,43 | 12,13 | 1,24 | 4.947,59 | 4 |
| | 50 | 0,27 | 0,23 | 1:1 | 0 | 32 | 0 | 0 | 6,89 | 0,5 | 55 | 0,64 | 8,25 | 1,82 | 7.275,87 | 4 |
| | 50 | 0,54 | 0,47 | 1:1 | 0 | 32 | 0 | 0 | 6,89 | 1,01 | 58 | 1,29 | 4,08 | 3,67 | 14.697,26 | 4 |
| | 60 | 0,18 | 0,16 | 1:1 | 0 | 29 | 0 | 0 | 6,89 | 0,34 | 38 | 0,43 | 12,13 | 1,24 | 4.947,59 | 4 |
| | 60 | 0,27 | 0,23 | 1:1 | 0 | 29 | 0 | 0 | 6,89 | 0,5 | 20 | 0,64 | 8,25 | 1,82 | 7.275,87 | 4 |
| | 60 | 0,54 | 0,47 | 1:1 | 0 | 29 | 0 | 0 | 6,89 | 1,01 | 18 | 1,29 | 4,08 | 3,67 | 14.697,26 | 4 |
| | 70 | 0,18 | 0,16 | 1:1 | 0 | 29 | 0 | 0 | 6,89 | 0,34 | 25 | 0,43 | 12,13 | 1,24 | 4.947,59 | 4 |
| | 70 | 0,27 | 0,23 | 1:1 | 0 | 29 | 0 | 0 | 6,89 | 0,5 | 20 | 0,64 | 8,25 | 1,82 | 7.275,87 | 4 |
| | 70 | 0,54 | 0,47 | 1:1 | 0 | 29 | 0 | 0 | 6,89 | 1,01 | 18 | 1,29 | 4,08 | 3,67 | 14.697,26 | 4 |
| | 80 | 0,18 | 0,16 | 1:1 | 0 | 29 | 0 | 0 | 6,89 | 0,34 | 25 | 0,43 | 12,13 | 1,24 | 4.947,59 | 4 |
| | 80 | 0,27 | 0,23 | 1:1 | 0 | 29 | 0 | 0 | 6,89 | 0,5 | 23 | 0,64 | 8,25 | 1,82 | 7.275,87 | 4 |
| | 80 | 0,54 | 0,47 | 1:1 | 0 | 29 | 0 | 0 | 6,89 | 1,01 | 15 | 1,29 | 4,08 | 3,67 | 14.697,26 | 4 |

Tabelle 6: Parameter zur $CO_2$-Beigabe

| A | B | C | D | E | F | G | H | I | J | K | L | M | N | O | P | Q | R |
|---|---|---|---|---|---|---|---|---|---|---|---|---|---|---|---|---|---|
| Werk-stoff | Temper-atur [°C] | Edukt 1 N-Me-thyl-Mor-pholin (NMM) [ml/min] | Edukt 2 Wasser-stoff per-oxid (H2O2) [ml/min] | Molver-hältnis Edukt 1: Edukt 2 [mol/mol] | CO2 in NMM [Mas se %] | Konzen-tration H2O2 [Masse %] | CO2 in H2O2 [Mas se %] | H3PO 4 in H2O2 [Mas se %] | Druc k [bar] | Durch-satz [ml/min] | Um-satz [%] | Aus-beute NMMO [%] | Fluidge-schwindigkeit [m/min] | hydrodyna-mische Ver-weilzeit im Reaktions-bereich - Reaktor-länge [min] | Reaktor-spezi-fische Ober-flächen belas-tung [l/m²h] | Reaktor - spezi-fische Volu-mens belas-tung [l/m³h] | Ober-fläche: Volu-men Ver-hältnis |
| 1.4404 | 60 | 2,7 | 2,42 | 1:1,1 | 0,5 | 29 | 0 | 0 | 20 | 5,12 | 84 | 75 | 6,52 | 0,69 | 21,73 | 86.922,38 | 4 |
|  | 60 | 2 | 1,79 | 1:1,1 | 0,5 | 29 | 0 | 0 | 20 | 3,79 | 86 | 75 | 4,83 | 0,93 | 16,09 | 64.342,94 | 4 |
|  | 60 | 1,6 | 1,43 | 1:1,1 | 0,5 | 29 | 0 | 0 | 20 | 3,03 | 86 | 74 | 3,86 | 1,17 | 12,86 | 51.440,39 | 4 |
|  | 60 | 0,8 | 0,72 | 1:1,1 | 0,5 | 29 | 0 | 0 | 20 | 1,52 | 87 | 74 | 1,94 | 2,33 | 6,45 | 25.805,08 | 4 |
|  | 60 | 0,5 | 0,45 | 1:1,1 | 0,5 | 32 | 0 | 0 | 20 | 0,95 | 83 | 81 | 1,21 | 3,72 | 4,03 | 16.128,18 | 4 |
|  | 60 | 4 | 3,58 | 1:1,1 | 0,5 | 32 | 0 | 0,1 | 20 | 7,58 | 69 | 68 | 9,65 | 0,47 | 32,17 | 128.685,87 | 4 |
|  | 60 | 2,7 | 2,42 | 1:1,1 | 0,5 | 29 | 0 | 0,1 | 20 | 5,12 | 83 | 81 | 6,52 | 0,69 | 21,73 | 86.922,38 | 4 |
|  | 60 | 2 | 1,79 | 1:1,1 | 0,5 | 29 | 0 | 0,1 | 20 | 3,79 | 90 | 90 | 4,83 | 0,93 | 16,09 | 64.342,94 | 4 |
|  | 60 | 0,8 | 0,72 | 1:1,1 | 0,5 | 29 | 0 | 0,1 | 20 | 1,52 | 92 | 90 | 1,94 | 2,33 | 6,45 | 25.805,08 | 4 |
|  | 60 | 0,5 | 0,45 | 1:1,1 | 0,5 | 29 | 0 | 0,1 | 20 | 0,95 | 91 | 90 | 1,21 | 3,72 | 4,03 | 16.128,18 | 4 |

Einfluss der $CO_2$ Konzentration auf die Reaktionsgeschwindigkeit

Der positive Effekt der Zugabe von $CO_2$ zur Reaktionslösung wird genauer untersucht. Um ihn zu quantifizieren, wurden Versuche bei unterschiedlichen Gew.% $CO_2$ (gelöst und bezogen auf das Amin) durchgeführt. Wie in Fig. 7 ersichtlich ist, steigt die Reaktionsgeschwindigkeit mit den gelösten Gew.% deutlich an. Parameter und Ergebnisse sind in Tabelle 6 angegeben.

Einfluss der Temperatur auf den Reaktionsfortschritt

Um eine möglichst hohe Reaktionsgeschwindigkeit, aber dennoch selektive Reaktion zu erreichen wurden Versuche bei 40-70°C durchgeführt (Fig. 8). Mit Temperaturen bis 60°C steigt der Umsatz und die Ausbeute an NMM massiv an. Allerdings verringert sich die Ausbeute bei 70°C, was auf ein Durchgehen der Reaktion und dadurch eine Zersetzung der Edukte und auch des Produkts zwischen 60 und 70°C hindeutet. Im Gegensatz zu diskontinuierlichen Betriebsweisen erlaubt das erfindungsgemäße kontinuierliche Verfahren bei größeren Produktionsmengen eine exakte Temperaturkontrolle, sodass die Temperiertemperaturen auch im Reaktionsfluid gut eingehalten werden können. Parameter und Ergebnisse sind in Tabelle 7 angegeben.

Einfluss der Konzentration des Wasserstoffperoxids auf die Produktionsrate

Um zu ermitteln welche Konzentration an Wasserstoffperoxid in der Reaktionslösung sinnvoll ist wurden identische Versuche mit 30 und mit 50 Gew.%igem Wasserstoffperoxid durchgeführt (Fig. 9). Die Verwendung von 50 Gew.%igem Wasserstoffperoxid erwies sich als günstiger. Zudem kann so konzentrierteres NMMNO gewonnen werden. Der Vorteil des erfindungsgemäßen Mikroreaktionssystems ist, dass der Einsatz von sehr konzentriertem Wasserstoffperoxid kein Problem hinsichtlich der Wärmetönung ist. Parameter und Ergebnisse sind in Tabelle 8 angegeben.

Tabelle 7: Temperaturvariationen

| A | B | C | D | E | F | G | H | I | J | K | L | M | N | O | P | Q | R |
|---|---|---|---|---|---|---|---|---|---|---|---|---|---|---|---|---|---|
| Werk-stoff | Temper-atur [°C] | Edukt 1 N-Me-thyl-Mor-pholin (NMM) [ml/min] | Edukt 2 Wasser-stoff per-oxid (H2O2) [ml/min] | Molver-hältnis Edukt 1 : Edukt 2 [mol/mol] | CO2 in NMM [Mas se %] | Konzen-tration H2O2 [Masse %] | CO2 in H2O2 [Mas se %] | H3PO 4 in H2O2 [Mas se %] | Druc k [bar] | Durch-satz [ml/min] | Um-satz [%] | Aus-beute NMMO [%] | Fluidge-schwindigkeit [m/min] | hydrodyna-mische Ver-weilzeit im Reaktions-bereich - Reaktor-länge [min] | Reaktor-spezi-fische Ober-flächen belas-tung [l/m²h] | Reaktor - spezi-fische Volu-mens belas-tung [l/m³h] | Ober-fläche: Volu-men Ver-hältnis |
| 1.4404 | 40 | 4,8 | 2,71 | 1:1,1 | 1 | 30 | 0 | 0,1 | 20 | 7,51 | 21 | 18 | 9,56 | 0,39 | 38,25 | 152.996,98 | 4 |
| | 40 | 3,2 | 1,81 | 1:1,1 | 1 | 30 | 0 | 0,1 | 20 | 5,01 | 27 | 26 | 6,38 | 0,59 | 25,52 | 102.065,89 | 4 |
| | 40 | 2,4 | 1,36 | 1:1,1 | 1 | 30 | 0 | 0,1 | 20 | 3,76 | 32 | 29 | 4,79 | 0,78 | 19,15 | 76.600,35 | 4 |
| | 40 | 1,2 | 0,68 | 1:1,1 | 1 | 30 | 0 | 0,1 | 20 | 1,88 | 38 | 37 | 2,39 | 1,57 | 9,58 | 38.300,18 | 4 |
| | 40 | 0,6 | 0,34 | 1:1,1 | 1 | 30 | 0 | 0,1 | 20 | 0,94 | 52 | 50 | 1,20 | 3,13 | 4,79 | 19.150,09 | 4 |
| | 40 | 0,47 | 0,27 | 1:1,1 | 1 | 30 | 0 | 0,1 | 20 | 0,74 | 57 | 55 | 0,94 | 3,98 | 3,77 | 15.075,60 | 4 |
| | 40 | 0,24 | 0,14 | 1:1,1 | 1 | 30 | 0 | 0,1 | 20 | 0,38 | 82 | 78 | 0,48 | 7,75 | 1,94 | 7.741,52 | 4 |
| | 50 | 4,8 | 2,71 | 1:1,1 | 1 | 30 | 0 | 0,1 | 20 | 7,51 | 30 | 30 | 9,56 | 0,39 | 38,25 | 152.996,98 | 4 |
| | 50 | 3,2 | 1,81 | 1:1,1 | 1 | 30 | 0 | 0,1 | 20 | 5,01 | 38 | 38 | 6,38 | 0,59 | 25,52 | 102.065,89 | 4 |
| | 50 | 2,4 | 1,36 | 1:1,1 | 1 | 30 | 0 | 0,1 | 20 | 3,76 | 43 | 42 | 4,79 | 0,78 | 19,15 | 76.600,35 | 4 |
| | 50 | 1,2 | 0,68 | 1:1,1 | 1 | 30 | 0 | 0,1 | 20 | 1,88 | 52 | 50 | 2,39 | 1,57 | 9,58 | 38.300,18 | 4 |
| | 50 | 0,6 | 0,34 | 1:1,1 | 1 | 30 | 0 | 0,1 | 20 | 0,94 | 69 | 68 | 1,20 | 3,13 | 4,79 | 19.150,09 | 4 |
| | 50 | 0,47 | 0,27 | 1:1,1 | 1 | 30 | 0 | 0,1 | 20 | 0,74 | 73 | 70 | 0,94 | 3,98 | 3,77 | 15.075,60 | 4 |
| | 50 | 0,24 | 0,14 | 1:1,1 | 1 | 30 | 0 | 0,1 | 20 | 0,38 | 90 | 90 | 0,48 | 7,75 | 1,94 | 7.741,52 | 4 |

EP 3 558 901 B1

| A | B | C | D | E | F | G | H | I | J | K | L | M | N | O | P | Q | R |
|---|---|---|---|---|---|---|---|---|---|---|---|---|---|---|---|---|---|
| Werkstoff | Temperatur [°C] | Edukt 1 N-Methyl-Morpholin (NMM) [ml/min] | Edukt 2 Wasserstoffperoxid (H2O2) [ml/min] | Molverhältnis Edukt 1 : Edukt 2 [mol/mol] | $CO_2$ in NMM [Masse %] | Konzentration H2O2 [Masse %] | $CO_2$ in H2O2 [Masse %] | H3PO4 in H2O2 [Masse %] | Druck [bar] | Durchsatz [ml/min] | Umsatz [%] | Ausbeute NMMO [%] | Fluidgeschwindigkeit [m/min] | hydrodynamische Verweilzeit im Reaktionsbereich - Reaktorlänge [min] | Reaktorspezifische Oberflächenbelastung [l/m²h] | Reaktor-spezifische Volumenbelastung [l/m³h] | Oberfläche: Volumen Verhältnis |
| | 60 | 4,1 | 3,5 | 1:1,1 | 1 | 30 | 0 | 0,1 | 20 | 7,6 | 82 | 80 | 9,68 | 0,39 | 38,71 | 154.830,49 | 4 |
| | 60 | 2,7 | 2,3 | 1:1,1 | 1 | 30 | 0 | 0,1 | 20 | 5 | 83 | 83 | 6,37 | 0,59 | 25,47 | 101.862,17 | 4 |
| | 60 | 2 | 1,71 | 1:1,1 | 1 | 30 | 0 | 0,1 | 20 | 3,71 | 86 | 85 | 4,72 | 0,79 | 18,90 | 75.581,73 | 4 |
| | 60 | 1 | 0,85 | 1:1,1 | 1 | 30 | 0 | 0,1 | 20 | 1,85 | 84 | 85 | 2,36 | 1,59 | 9,42 | 37.689,00 | 4 |
| | 60 | 0,5 | 0,43 | 1:1,1 | 1 | 30 | 0 | 0,1 | 20 | 0,93 | 90 | 90 | 1,18 | 3,17 | 4,74 | 18.946,36 | 4 |
| | 60 | 0,4 | 0,34 | 1:1,1 | 1 | 30 | 0 | 0,1 | 20 | 0,74 | 88 | 87 | 0,94 | 3,98 | 3,77 | 15.075,60 | 4 |
| | 60 | 0,21 | 0,18 | 1:1,1 | 1 | 30 | 0 | 0,1 | 20 | 0,39 | 96 | 85 | 0,50 | 7,55 | 1,99 | 7.945,25 | 4 |
| | 70 | 4,8 | 2,71 | 1:1,1 | 1 | 30 | 0 | 0,1 | 20 | 7,51 | 35 | 30 | 9,56 | 0,39 | 38,25 | 152.996,98 | 4 |
| | 70 | 3,2 | 1,81 | 1:1,1 | 1 | 30 | 0 | 0,1 | 20 | 5,01 | 25 | 22 | 6,38 | 0,59 | 25,52 | 102.065,89 | 4 |
| | 70 | 2,4 | 1,36 | 1:1,1 | 1 | 30 | 0 | 0,1 | 20 | 3,76 | 28 | 15 | 4,79 | 0,78 | 19,15 | 76.600,35 | 4 |
| | 70 | 1,2 | 0,68 | 1:1,1 | 1 | 30 | 0 | 0,1 | 20 | 1,88 | 25 | 23 | 2,39 | 1,57 | 9,58 | 38.300,18 | 4 |
| | 70 | 0,6 | 0,34 | 1:1,1 | 1 | 30 | 0 | 0,1 | 20 | 0,94 | 33 | 24 | 1,20 | 3,13 | 4,79 | 19.150,09 | 4 |
| | 70 | 0,47 | 0,27 | 1:1,1 | 1 | 30 | 0 | 0,1 | 20 | 0,74 | 28 | 22 | 0,94 | 3,98 | 3,77 | 15.075,60 | 4 |
| | 70 | 0,24 | 0,14 | 1:1,1 | 1 | 30 | 0 | 0,1 | 20 | 0,38 | 25 | 16 | 0,48 | 7,75 | 1,94 | 7.741,52 | 4 |

Tabelle 8: Parameter zur $H_2O_2$-Beigabe

| A | B | C | D | E | F | G | H | I | J | K | L | M | N | O | P | Q | R |
|---|---|---|---|---|---|---|---|---|---|---|---|---|---|---|---|---|---|
| Werkstoff | Temperatur [°C] | Edukt 1 N-Methyl-Morpholin (NMM) [ml/min] | Edukt 2 Wasserstoffperoxid (H2O2) [ml/min] | Molverhältnis Edukt 1 : Edukt 2 [mol/mol] | CO2 in NMM [Masse %] | Konzentration H2O2 [Masse %] | CO2 in H2O2 [Masse %] | H3PO4 in H2O2 [Masse %] | Druck [bar] | Durchsatz [ml/min] | Umsatz [%] | Ausbeute NMMO [%] | Fluidgeschwindigkeit [m/min] | hydrodynamische Verweilzeit im Reaktionsbereich - Reaktorlänge [min] | Reaktorspezifische Oberflächenbelastung [l/m²h] | Reaktor-spezifische Volumensbelastung [l/m³h] | Oberfäche: Volumen Verhältnis |
| 1.4404 | 60 | 4,8 | 2,71 | 1:1,1 | 1 | 30 | 0 | 0,1 | 20 | 7,51 | 39 | 38 | 9,56 | 0,39 | 38,2 | 152.997,0 | 4 |
| | 60 | 3,2 | 1,81 | 1:1,1 | 1 | 30 | 0 | 0,1 | 20 | 5,01 | 47 | 45 | 6,38 | 0,59 | 25,5 | 102.065,9 | 4 |
| | 60 | 2,4 | 1,36 | 1:1,1 | 1 | 30 | 0 | 0,1 | 20 | 3,76 | 51 | 45 | 4,79 | 0,78 | 19,2 | 76.600,4 | 4 |
| | 60 | 1,2 | 0,68 | 1:1,1 | 1 | 30 | 0 | 0,1 | 20 | 1,88 | 63 | 60 | 2,39 | 1,57 | 9,6 | 38.300,2 | 4 |
| | 60 | 0,6 | 0,34 | 1:1,1 | 1 | 30 | 0 | 0,1 | 20 | 0,94 | 79 | 75 | 1,20 | 3,13 | 4,8 | 19.150,1 | 4 |
| | 60 | 0,47 | 0,27 | 1:1,1 | 1 | 30 | 0 | 0,1 | 20 | 0,74 | 82 | 80 | 0,94 | 3,98 | 3,8 | 15.075,6 | 4 |
| | 60 | 0,24 | 0,14 | 1:1,1 | 1 | 30 | 0 | 0,1 | 20 | 0,38 | 93 | 90 | 0,48 | 7,75 | 1,9 | 7.741,5 | 4 |
| | 60 | 0,17 | 0,1 | 1:1,1 | 1 | 30 | 0 | 0,1 | 20 | 0,27 | 98 | 95 | 0,34 | 10,91 | 1,4 | 5.500,6 | 4 |
| | 60 | 4,8 | 2,71 | 1:1,1 | 1 | 50 | 0 | 0,1 | 20 | 7,51 | 47 | 45 | 9,56 | 0,39 | 38,2 | 152.997,0 | 4 |
| | 60 | 3,2 | 1,81 | 1:1,1 | 1 | 50 | 0 | 0,1 | 20 | 5,01 | 57 | 55 | 6,38 | 0,59 | 25,5 | 102.065,9 | 4 |
| | 60 | 2,4 | 1,36 | 1:1,1 | 1 | 50 | 0 | 0,1 | 20 | 3,76 | 63 | 60 | 4,79 | 0,78 | 19,2 | 76.600,4 | 4 |
| | 60 | 1,2 | 0,68 | 1:1,1 | 1 | 50 | 0 | 0,1 | 20 | 1,88 | 72 | 70 | 2,39 | 1,57 | 9,6 | 38.300,2 | 4 |
| | 60 | 0,6 | 0,34 | 1:1,1 | 1 | 50 | 0 | 0,1 | 20 | 0,94 | 86 | 84 | 1,20 | 3,13 | 4,8 | 19.150,1 | 4 |
| | 60 | 0,47 | 0,27 | 1:1,1 | 1 | 50 | 0 | 0,1 | 20 | 0,74 | 87 | 84 | 0,94 | 3,98 | 3,8 | 15.075,6 | 4 |
| | 60 | 0,24 | 0,14 | 1:1,1 | 1 | 50 | 0 | 0,1 | 20 | 0,38 | 97 | 97 | 0,48 | 7,75 | 1,9 | 7.741,5 | 4 |
| | 60 | 0,17 | 0,1 | 1:1,1 | 1 | 50 | 0 | 0,1 | 20 | 0,27 | 97 | 95 | 0,34 | 10,91 | 1,4 | 5.500,6 | 4 |
| | 60 | 4,8 | 2,71 | 1:1,1 | 0,5 | 50 | 0 | 0,1 | 20 | 7,51 | 38 | 34 | 9,56 | 0,39 | 38,2 | 152.997,0 | 4 |
| | 60 | 3,2 | 1,81 | 1:1,1 | 0,5 | 50 | 0 | 0,1 | 20 | 5,01 | 34 | 33 | 6,38 | 0,59 | 25,5 | 102.065,9 | 4 |
| | 60 | 2,4 | 1,36 | 1:1,1 | 0,5 | 50 | 0 | 0,1 | 20 | 3,76 | 54 | 53 | 4,79 | 0,78 | 19,2 | 76.600,4 | 4 |
| | 60 | 1,2 | 0,68 | 1:1,1 | 0,5 | 50 | 0 | 0,1 | 20 | 1,88 | 60 | 42 | 2,39 | 1,57 | 9,6 | 38.300,2 | 4 |

EP 3 558 901 B1

(fortgesetzt)

| A | B | C | D | E | F | G | H | I | J | K | L | M | N | O | P | Q | R |
|---|---|---|---|---|---|---|---|---|---|---|---|---|---|---|---|---|---|
| Werk-stoff | Temper-atur [°C] | Edukt 1 N-Me-thyl-Mor-pholin (NMM) [ml/min] | Edukt 2 Wasser-stoff per-oxid (H2O2) [ml/min] | Molver-hältnis Edukt 1 : Edukt 2 [mol/mol] | CO2 in NMM [Masse %] | Konzentration H2O2 [Masse %] | CO2 in H2O2 [Masse %] | H3PO4 in H2O2 [Masse %] | Druck [bar] | Durch-satz [ml/min] | Um-satz [%] | Aus-beute NMMO [%] | Fluidge-schwindigkeit [m/min] | hydrodyna-mische Ver-weilzeit im Reaktions-bereich - Reaktor-länge [min] | Reaktor-spezi-fische Ober-flächen belas-tung [l/m$^2$h] | Reaktor-spezi-fische Volu-mens belas-tung [l/m$^3$h] | Ober-fäche: Volu-men Verhält-nis |
| | 60 | 0,6 | 0,34 | 1:1,1 | 0,5 | 50 | 0 | 0,1 | 20 | 0,94 | 70 | 69 | 1,20 | 3,13 | 4,8 | 19.150,1 | 4 |
| | 60 | 0,47 | 0,27 | 1:1,1 | 0,5 | 50 | 0 | 0,1 | 20 | 0,74 | 81 | 80 | 0,94 | 3,98 | 3,8 | 15.075,6 | 4 |
| | 60 | 0,24 | 0,14 | 1:1,1 | 0,5 | 50 | 0 | 0,1 | 20 | 0,38 | 90 | 90 | 0,48 | 7,75 | 1,9 | 7.741,5 | 4 |
| | 60 | 0,17 | 0,1 | 1:1,1 | 0,5 | 50 | 0 | 0,1 | 20 | 0,27 | 99 | 95 | 0,34 | 10,91 | 1,4 | 5.500,6 | 4 |

Großtechnischer Reaktor

Es wurden weitere Experimente mit den gleichen Chemikalien und analoger Versuchsdurchführung wie in den bisherigen Beispielen nur mit größerem Reaktor durchgeführt.

Das Design des größeren Reaktors ist ähnlich dem oben beschriebenen Reaktordesign mit folgenden Änderungen. Die Länge der verwendeten Rohre betrug 2,8 m mit einem Innendurchmesser von 1,5 bis 4 mm, mit und ohne Quetschungen. Die Anzahl der verwendeten Rohre betrug 40. Die erzielte Produktionskapazität in diesem Experiment war von 1 kg/h bis 3 kg/h NMMNO bezogen auf 100%iges NMMNO. Durch diese Produktionskapazität war es nötig, anstatt des Heizthermostats (siehe Reaktorskizze (2) - (Lauda: ECO SILVER RE415)) einen Kühler zu installieren.

Es wurde bei leichtem Überdruck (0,3 barü) 1 Gew-% $CO_2$ in NMM gelöst und anschließend das $NMM/CO_2$ in den Vorlagebehälter (3) überführt. Nun wurde 0,1 Gew.-% $H_3PO_4$ in die $H_2O_2$ Lösung gegeben und in den Vorlagebehälter (4) geleert. Die weitere Vorgehensweise ist ident zu den vorhin beschriebenen Beispielen.

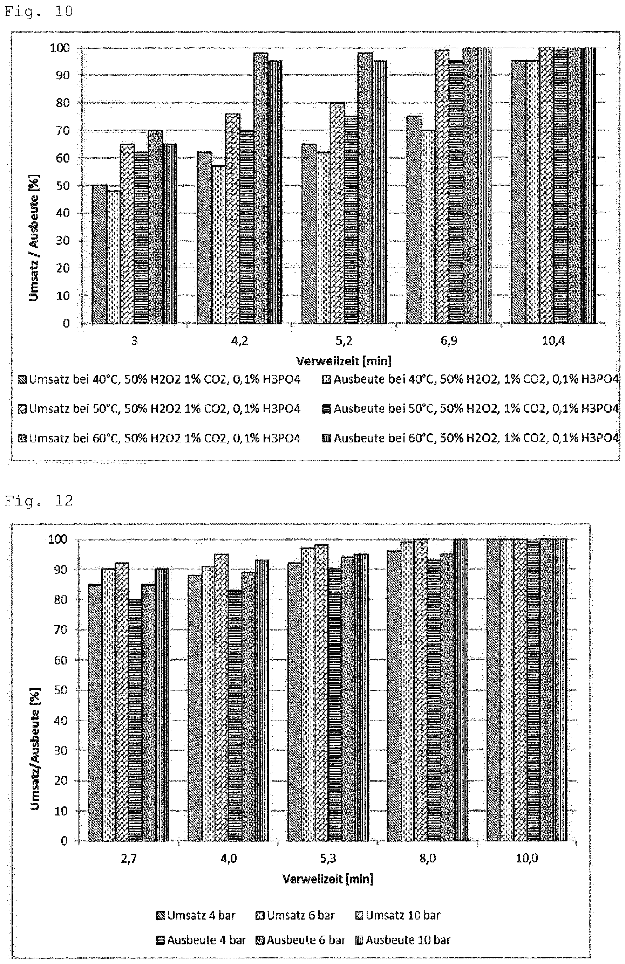

In diesem Experiment wurde versucht eine möglichst hohe Produktionskapazität durch den größeren Reaktor und durch größere Volumenströme zu erzielen. Ergebnisse sind in Fig. 10 und in Tabelle 9 gezeigt.

Tabelle 9: Großtechnischer Reaktor

| A | B | C | D | E | F | G | H | I | J | K | L | M | N | O | P | Q | R |
|---|---|---|---|---|---|---|---|---|---|---|---|---|---|---|---|---|---|
| Werkstoff | Temperatur [°C] | Edukt 1 N-Methyl-Morpholin (NMM) [ml/min] | Edukt 2 Wasserstoff peroxid (H2O2) [ml/min] | Molverhältnis Edukt 1 : Edukt 2 [mol/mol] | CO2 in NMM [Masse %] | Konzentration H2O2 [Masse %] | CO2 in H2O2 [Masse %] | H3PO4 in H2O2 [Masse %] | Druck [bar] | Durchsatz [ml/min] | Umsatz [%] | Ausbeute NMMO [%] | max. Fluidgeschwindigkeit [m/min] | hydrodynamische Verweilzeit im Reaktionsbereich [min] | Reaktor-spezifische Oberflächen belastung [l/m²h] | Reaktor-spezifische Volumens belastung [l/m³h] | Oberfläche:Volumen Verhältnis |
| 1.4404 | 40 | 117,3 | 66,0 | 1:1,0 | 1 | 50,75 | 0 | 0,1 | 103 | 183,3 | 50 | 48 | 103,7 | 0,8 | 56,87 | 74445 | |
| | 40 | 83,8 | 47,1 | 1:1,1 | 1 | 50,75 | 0 | 0,1 | 68 | 130,9 | 62 | 57 | 74,1 | 1,1 | 40,61 | 53163 | |
| | 40 | 67 | 37,7 | 1:1,1 | 1 | 50,75 | 0 | 0,1 | 55 | 104,7 | 65 | 62 | 59,2 | 1,4 | 32,48 | 42523 | |
| | 40 | 50,3 | 28,3 | 1:1,1 | 1 | 50,75 | 0 | 0,1 | 43 | 78,6 | 75 | 70 | 44,5 | 1,9 | 24,39 | 31922 | |
| | 40 | 33,5 | 18,8 | 1:1,1 | 1 | 50,75 | 0 | 0,1 | 34 | 52,3 | 95 | 95 | 29,6 | 2,8 | 16,23 | 21241 | |
| | 50 | 117,3 | 66,0 | 1:1,0 | 1 | 50,75 | 0 | 0,1 | 95 | 183,3 | 65 | 62 | 103,7 | 0,8 | 56,87 | 74445 | |
| | 50 | 83,8 | 47,1 | 1:1,1 | 1 | 50,75 | 0 | 0,1 | 65 | 130,9 | 76 | 70 | 74,1 | 1,1 | 40,61 | 53163 | |
| | 50 | 67 | 37,7 | 1:1,1 | 1 | 50,75 | 0 | 0,1 | 52 | 104,7 | 80 | 75 | 59,2 | 1,4 | 32,48 | 42523 | |
| | 50 | 50,3 | 28,3 | 1:1,1 | 1 | 50,75 | 0 | 0,1 | 46 | 78,6 | 99 | 95 | 44,5 | 1,9 | 24,39 | 31922 | |
| | 50 | 33,5 | 18,8 | 1:1,1 | 1 | 50,75 | 0 | 0,1 | 32 | 52,3 | 100 | 99 | 29,6 | 2,8 | 16,23 | 21241 | |
| | 60 | 117,3 | 66,0 | 1:1,0 | 1 | 50,75 | 0 | 0,1 | 92 | 183,3 | 70 | 65 | 103,7 | 0,8 | 56,87 | 74445 | |
| | 60 | 83,8 | 47,1 | 1:1,1 | 1 | 50,75 | 0 | 0,1 | 65 | 130,9 | 98 | 95 | 74,1 | 1,1 | 40,61 | 53163 | |
| | 60 | 67 | 37,7 | 1:1,1 | 1 | 50,75 | 0 | 0,1 | 50 | 104,7 | 98 | 95 | 59,2 | 1,4 | 32,48 | 42523 | |
| | 60 | 50,3 | 28,3 | 1:1,1 | 1 | 50,75 | 0 | 0,1 | 41 | 78,6 | 100 | 100 | 44,5 | 1,9 | 24,39 | 31922 | |
| | 60 | 33,5 | 18,8 | 1:1,1 | 1 | 50,75 | 0 | 0,1 | 28 | 52,3 | 100 | 100 | 29,6 | 2,8 | 16,23 | 21241 | |

1,5-4 mm verwendet. Dies entspricht einer Verringerung des Oberfläche:Volumen Verhältnisses von 2,7 auf 1 über die Reaktorlänge, wobei es bis zu Umsätzen von 50% sicher >2 ist.

## Reaktionssystem nach Fig. 11

### CO$_2$ Sättigung direkt im Mikroreaktor

Der Versuch wurde wie zuvor beschrieben mit dem kleinen Reaktor durchgeführt mit dem Unterschied, dass das Edukt 1 NMM unverdünnt 99%ig über eine computergesteuerte Pumpe (5) aus dem Vorratsbehälter (3) in den Mikroreaktor (1) gefördert wird und mit Kühlwasser auf ca. 20°C temperiert wird. Die maximal lösliche Gasmenge, welche aus den Löslichkeitsversuchen ermittelt wurde, wird über ein Dosierventil (14) aus der Gasflasche (15) in den Mikroreaktor geleitet. Der Druck im Abschnitt der CO$_2$ Sättigung wird auf [A] bar geregelt.

Es wurden zwei Heizthermostate (2, 16) ECO SILVER RE415 der Firma Lauda eingesetzt um den Mikroreaktor auf T1=20 °C bzw. T2=65 °C zu temperieren. Als Kühl- und Heizmedium wurde ein Gemisch aus Wasser/Ethylenglycol im Volumensverhältnis 1:1 eingesetzt.

Im Wasserstoffperoxid Vorratsbehälter (4) wird das Edukt 2, Wasserstoffperoxid, mit 0,1 Gew.-% H$_3$PO$_4$ versetzt, vorgelagert und mit einer Pumpe (6) in den Mikroreaktor (1) gefördert. Nach dem Sättigungsrohr (7) und Vorwärmrohr (8) werden die Edukte über ein Mischmodul T3 (9) zusammengeführt. Im Mischmodul wird ein statischer Mischer (z.B. Mischplättchen) eingesetzt, gemäß WO 2010/055034. Anschließend wird das Reaktionsgemisch in die Reaktionsrohre (10) geleitet, wo dann die Reaktion stattfindet. Zur Erhöhung und Regelung des Drucks im Reaktor wurde nach dem Reaktoraustritt ein Druckhalteventil (11) mit 20 bar eingesetzt. Der Einbau des Druckhalteventils sollte die Zerfalls- und Nebenreaktionen möglichst unterdrücken, da es im Zuge dieser unerwünschten Reaktionen zur Gasentwicklung kommt.

Das gewünschte Produkt wird nach einer Infrarotmessung (13) in einem Gefäß mit Rührwerk (12) aufgefangen. Überschüssiges Wasserstoffperoxid wird durch das Überleiten der Reaktionslösung über immobilisiertes MnO$_2$ zerstört.

**Tabelle 10: Edukte der separaten CO$_2$-Zufuhr:**

|  | Edukt 1 | Edukt 2 | Edukt 3 |
|---|---|---|---|
| Volumenstrom | [B] ml/min | [C] ml/min | [D] Gew.-% bezogen auf Edukt 1 |
| Molares Verhältnis | | 1:1,1 | |
| Zusammensetzung | 99 Vol.-%iges N-Metyhlmorpholin | 50 Gew.-%iges Wasserstoffperoxid mit 0,1 Gew.-% H$_3$PO$_4$ | CO$_2$ |

Durchsätze von [E] ml/min mit Umsätzen von [F]% und Ausbeuten von [G]% konnten erreicht werden. Ergebnisse sind in Fig. 12 und Tab. 12 gezeigt.

Tabelle 11 Parameter und Einheiten:

| | |
|---|---|
| Fluidgeschwindigkeit | [H] m/min |
| Hydrodynamische Verweilzeit im Reaktionsbereich | [I] min |
| Reaktorspezifische Oberflächenbelastung | [J] L/m$^2$h |
| Reaktorspezifische Volumensbelastung | [K] L/m$^3$h |
| Oberflächen:Volumen Verhältnis | [L] |

Tabelle 12: Ergebnisse der separaten $CO_2$-Zufuhr:

| A | B | C | D | E | F | G | H | I | J | K | L |
|---|---|---|---|---|---|---|---|---|---|---|---|
| Druck im Sättigungs-Abschnitt [bar] | Edukt 1 N-Methyl-Morpholin (NMM) [ml/min] | Edukt 2 Wasserstoff peroxid (H2O2) [ml/min] | CO2 in NMM [Masse %] | Durchsatz [ml/min] | Umsatz [%] | Ausbeute NMMO [%] | max. Fluidgeschwindigkeit [m/min] | hydrodynamische Verweilzeit im Reaktionsbereich [min] | Reaktor-spezifische Oberflächen belastung [l/m²h] | Reaktor-spezifische Volumens belastung [l/m³h] | Oberfläche: Volum en Verhältnis |
| 4 | 5,03 | 2,83 | 5 | 7,86 | 85 | 80 | 10,0 | 2,7 | 5,56 | 22457 | 4 |
| 4 | 3,35 | 1,88 | 5 | 5,23 | 88 | 83 | 6,7 | 4,0 | 3,70 | 14943 | 4 |
| 4 | 2,51 | 1,41 | 5 | 3,92 | 92 | 90 | 5,0 | 5,3 | 2,77 | 11200 | 4 |
| 4 | 1,68 | 0,9 | 5 | 2,6 | 96 | 93 | 3,3 | 8,0 | 1,85 | 7486 | 4 |
| 4 | 1,34 | 0,75 | 5 | 2,09 | 100 | 99 | 2,7 | 10,0 | 1,48 | 5971 | 4 |
| 6 | 5,03 | 2,83 | 7,5 | 7,86 | 90 | 85 | 10,0 | 2,7 | 5,56 | 22457 | 4 |
| 6 | 3,35 | 1,88 | 7,5 | 5,23 | 91 | 89 | 6,7 | 4,0 | 3,70 | 14943 | 4 |
| 6 | 2,51 | 1,41 | 7,5 | 3,92 | 97 | 94 | 5,0 | 5,3 | 2,77 | 11200 | 4 |
| 6 | 1,68 | 0,9 | 7,5 | 2,62 | 99 | 95 | 3,3 | 8,0 | 1,85 | 7486 | 4 |
| 6 | 1,34 | 0,75 | 7,5 | 2,09 | 100 | 100 | 2,7 | 10,0 | 1,48 | 5971 | 4 |
| 10 | 5,03 | 2,83 | 14 | 7,86 | 92 | 90 | 10,0 | 2,7 | 5,56 | 22457 | 4 |
| 10 | 3,35 | 1,88 | 14 | 5,23 | 95 | 93 | 6,7 | 4,0 | 3,70 | 14943 | 4 |
| 10 | 2,51 | 1,41 | 14 | 3,92 | 98 | 95 | 5,0 | 5,3 | 2,77 | 11200 | 4 |
| 10 | 1,68 | 0,9 | 14 | 2,62 | 100 | 100 | 3,3 | 8,0 | 1,85 | 7486 | 4 |
| 10 | 1,34 | 0,75 | 14 | 2,09 | 100 | 100 | 2,7 | 10,0 | 1,48 | 5971 | 4 |

**Patentansprüche**

1. Verfahren zur Herstellung eines Aminoxids durch Oxidation eines tertiären Amins in einem Reaktor mit einem länglichen Hohlkörper unter kontinuierlicher Einführung von tertiärem Amin in einem Reaktionsfluid und Ausführung von Aminoxid, wobei i) im Hohlkörper ein Oberfläche zu Volumenverhältnis von 0,5 m$^2$/m$^3$ oder mehr zumindest auf einer Länge des Hohlkörpers, in der das Amin zu 50% (mol.-%) oxidiert wird, vorhanden ist;
ii) im Hohlkörper eine spezifische Oberflächenbelastung von 1 l/m$^2$h bis 40 l/m$^2$h vorhanden ist; und/oder
iii) im Hohlkörper eine spezifische Volumenbelastung von 1.000 l/m$^3$h bis 30.000 l/m$^3$h vorhanden ist; und wobei im Hohlkörper das Reaktionsfluid mit laminarer Strömung geführt wird.

2. Verfahren nach Anspruch 1, **dadurch gekennzeichnet, dass** das tertiäre Amin vorzugsweise in einem Molverhältnis von 1:0,9 bis 1:1,3, vorzugsweise 1:1 bis 1:1,1 zum Wasserstoffperoxid als Oxidationsmittel eingesetzt wird.

3. Verfahren nach Anspruch 1 oder 2, **dadurch gekennzeichnet, dass** das Aminoxid N-Methyl-Morpholin-N-oxid und das tertiäre Amin N-Methylmorpholin sind.

4. Verfahren nach einem der Ansprüche 1 bis 3, **dadurch gekennzeichnet, dass** eine Strömungsgeschwindigkeit des Reaktionsfluids im Hohlkörper auf eine Verweilzeit des tertiären Amins von 0,4 Minuten bis 14 Minuten, bevorzugt 0,6 Minuten bis 8 Minuten, eingestellt wird.

5. Verfahren nach einem der Ansprüche 1 bis 4, **dadurch gekennzeichnet, dass** die Strömungsgeschwindigkeit des Reaktionsfluids 0,1 m/min bis 200 m/min, bevorzugt 10 m/min bis 160 m/min, am meisten bevorzugt 10 m/min bis 120 m/min, ist.

6. Verfahren nach einem der Ansprüche 1 bis 5, **dadurch gekennzeichnet, dass** die Reaktionsfluidtemperatur im Hohlkörper 20°C bis 70°C, bevorzugt 40°C bis 65°C, am meisten bevorzugt von 55°C bis 65°C, ist.

7. Verfahren nach einem der Ansprüche 1 bis 6, **dadurch gekennzeichnet, dass** der Druck im Hohlkörper 1 bar bis 200 bar, bevorzugt 5 bar bis 100 bar, am meisten bevorzugt 50 bar bis 100 bar, ist.

8. Verfahren nach einem der Ansprüche 1 bis 7, **dadurch gekennzeichnet, dass** der Innendurchmesser eines Hohlkörpers, vorzugsweise eines Rohres, des Reaktors 0,25 mm bis 10 mm ist, vorzugsweise 0,5 mm bis 6 mm, insbesondere bevorzugt 0,8 mm bis 4 mm, ist.

9. Verfahren nach einem der Ansprüche 1 bis 8, **dadurch gekennzeichnet, dass** die Länge eines Hohlkörpers, vorzugsweise Rohres, des Reaktors 0,5 m bis 20 m, bevorzugt 1 m bis 10 m, insbesondere 2 m bis 5 m, ist.

10. Verfahren nach einem der Ansprüche 1 bis 9, **dadurch gekennzeichnet, dass** eine Innenwand eines Hohlkörpers, vorzugsweise Rohres, des Reaktors ein oder mehrere Metalle enthält, vorzugsweise ausgewählt aus Fe, Cr, Ni, Mo, Co, Mn, Nb, Au, Pt, Ti oder Mischungen hiervon, wobei insbesondere bevorzugt eine Innenwand eines Hohlkörpers, vorzugsweise Rohres, aus austenitischem Stahl ist.

11. Verfahren nach einem der Ansprüche 1 bis 10, **dadurch gekennzeichnet, dass** das tertiäre Amin in einer Konzentration von 40 % bis 100%, vorzugsweise von 50% bis 99,5%, insbesondere 60 bis 99%, speziell bevorzugt 70% bis 99%, in den Hohlkörper zugeführt wird (alle % in vol.-%).

12. Verfahren nach einem der Ansprüche 1 bis 11, **dadurch gekennzeichnet, dass** ein Oxidationsmittel, vorzugsweise Wasserstoffperoxid, in einer Konzentration von 5 % bis 80%, vorzugsweise von 10% bis 70%, insbesondere 20 bis 60%, speziell bevorzugt 30% bis 55%, in den Hohlkörper zugeführt wird (alle % in gew.-%).

13. Verfahren nach einem der Ansprüche 1 bis 12, **dadurch gekennzeichnet, dass** das Reaktionsfluid bei einer Bodensteinzahle von größer 10, bevorzugt von 20 bis 200, gemäß der Formel

$$Bo = u * L/D_{ax},$$

worin Bo die Bodensteinzahl ist, u die Strömungsgeschwindigkeit des Reaktionsfluids, L die Länge des Hohlkörpers, vorzugsweise Rohres, des Reaktors und $D_{ax}$ der axiale Dispersionsstrom des Reaktionsfluids ist, durch den Hohlkörper befördert wird.

14. Verfahren nach einem der Ansprüche 1 bis 13, **dadurch gekennzeichnet, dass** $CO_2$, vorzugsweise in einer Menge von 0,5 Gew.-% bis 20 Gew.-% bezogen auf das tertiäre Amin, in den Hohlkörper zugeführt wird.

15. Verfahren nach einem der Ansprüche 1 bis 14, **dadurch gekennzeichnet, dass** ein Metallkomplexbildner, vorzugsweise ein Phosphat, dem Reaktionsfluid zugefügt wird, vorzugsweise in einer Menge von 0,01% bis 3%, vorzugsweise 0,03% bis 2%, speziell bevorzugt 0,05% bis 1,5% (alle Gew.-%).

16. Verfahren nach einem der Ansprüche 1 bis 15, **dadurch gekennzeichnet, dass** die Oxidation des tertiären Amins mit einem Oxidationsmittel durchgeführt wird und überschüssiges Oxidationsmittel abgebaut oder entfernt wird.

17. Verfahren nach einem der Ansprüche 1 bis 16, **dadurch gekennzeichnet, dass** das Aminoxid auf eine Konzentration zum Auflösen von Zellulose gebracht wird und optional weiters Zellulose darin aufgelöst wird und optional weiters die aufgelöste Zellulose in Endlosformkörper geformt wird und die Zellulose der Formkörper gefällt wird.

18. Verfahren nach einem der Ansprüche 1 bis 17, **dadurch gekennzeichnet, dass** das Reaktionsfluid in Strömung bei einer Reynolds-Zahl von 2300 oder kleiner durch den Hohlkörper geführt wird, vorzugsweise bei einer Reynolds-Zahl von 1000 oder kleiner, insbesondere bevorzugt bei einer Reynolds-Zahl von 1 bis 100.

**Claims**

1. A method for producing an amine oxide by oxidation of a tertiary amine in a reactor with an elongate hollow body, under continuous introduction of tertiary amine in a reaction fluid and export of amine oxide, wherein

   i) a surface-to-volume ratio of 0.5 $m^2/m^3$ or more is provided in the hollow body, at least over a length of the hollow body in which the amine is oxidised to an extent of 50 % (mol %);
   ii) a specific surface load of 1 $l/m^2h$ to 40 $l/m^2h$ is provided in the hollow body; and/or
   iii) a specific volume load of 1,000 $l/m^3h$ to 30,000 $l/m^3h$ is provided in the hollow body; and wherein the reaction fluid is guided in the hollow body with a laminar flow.

2. The method according to claim 1, **characterised in that** the tertiary amine is preferably used in a molar ratio of 1:0.9 to 1:1.3, preferably 1:1 to 1:1.1, to hydrogen peroxide as oxidant.

3. The method according to claim 1 or 2, **characterised in that** the amine oxide is N-methylmorpholine N-oxide and the tertiary amine is N-methylmorpholine.

4. The method according to any one of claims 1 to 3, **characterised in that** a flow velocity of the reaction fluid in the hollow body is set to a residence time of the tertiary amine of 0.4 minutes to 14 minutes, preferably 0.6 minutes to 8 minutes.

5. The method according to any one of claims 1 to 4, **characterised in that** the flow velocity of the reaction fluid is 0.1 m/min to 200 m/min, preferably 10 m/min to 160 m/min, most preferably 10 m/min to 120 m/min.

6. The method according to any one of claims 1 to 5, **characterised in that** the reaction fluid temperature in the hollow body is 20 °C to 70 °C, preferably 40 °C to 65 °C, most preferably from 55 °C to 65 °C.

7. The method according to any one of claims 1 to 6, **characterised in that** the pressure in the hollow body is 1 bar to 200 bar, preferably 5 bar to 100 bar, most preferably 50 bar to 100 bar.

8. The method according to any one of claims 1 to 7, **characterised in that** the inner diameter of a hollow body, preferably a tube, of the reactor is 0.25 mm to 10 mm, preferably 0.5 mm to 6 mm, particularly preferably 0.8 mm to 4 mm.

9. The method according to any one of claims 1 to 8, **characterised in that** the length of a hollow body, preferably tube, of the reactor is 0.5 m to 20 m, preferably 1 m to 10 m, in particular 2 m to 5 m.

10. The method according to any one of claims 1 to 9, **characterised in that** an inner wall of a hollow body, preferably tube, of the reactor contains one or more metals, preferably selected from Fe, Cr, Ni, Mo, Co, Mn, Nb, Au, Pt, Ti or mixtures hereof, wherein an inner wall of a hollow body, preferably tube, is particularly preferably made of austenitic steel.

11. The method according to any one of claims 1 to 10, **characterised in that** the tertiary amine is fed into the hollow body in a concentration of 40 % to 100 %, preferably from 50 % to 99.5 %, in particular 60 to 99 %, especially preferably 70 % to 99 % (all % in vol. %).

12. The method according to any one of claims 1 to 11, **characterised in that** an oxidant, preferably hydrogen peroxide, is fed into the hollow body in a concentration of 5 % to 80 %, preferably from 10 % to 70 %, in particular 20 to 60 %, especially preferably 30 % to 55 % (all % in % by weight).

13. The method according to any one of claims 1 to 12, **characterised in that** the reaction fluid is conveyed through the hollow body at a Bodenstein number of greater than 10, preferably from 20 to 200, according to the formula

$$Bo = u * L/D_{ax},$$

in which Bo is the Bodenstein number, u is the flow velocity of the reaction fluid, L is the length of the hollow body, preferably tube, and $D_{ax}$ is the axial dispersion flow of the reaction fluid.

14. The method according to any one of claims 1 to 13, **characterised in that** $CO_2$ is fed into the hollow body, preferably in an amount of 0.5 % by weight to 20 % by weight, in relation to the tertiary amine.

15. The method according to any one of claims 1 to 14, **characterised in that** a metal complexing agent, preferably a phosphate, is added to the reaction fluid, preferably in an amount of 0.01 % to 3 %, preferably 0.03 % to 2 %, especially preferably 0.05 % to 1.5 % (all % by weight).

16. The method according to any one of claims 1 to 15, **characterised in that** the oxidation of the tertiary amine is performed with an oxidant and excess oxidant is broken down or removed.

17. The method according to any one of claims 1 to 16, **characterised in that** the amine oxide is brought to a concentration for the dissolution of cellulose and optionally furthermore cellulose is dissolved therein, and optionally furthermore the dissolved cellulose is shaped into continuous shaped bodies and the cellulose of the shaped bodies is precipitated.

18. The method according to any one of claims 1 to 17, **characterised in that** the reaction fluid is guided in a flow through the hollow body at a Reynolds number of 2300 or less, preferably at a Reynolds number of 1000 or less, particularly preferably at a Reynolds number of 1 to 100.

**Revendications**

1. Procédé de préparation d'un oxyde d'amine par oxydation d'une amine tertiaire dans un réacteur comportant une partie creuse oblongue, avec introduction continue d'une amine tertiaire dans un fluide réactionnel et évacuation d'oxyde d'amine, procédé dans lequel

i) la partie creuse présente un rapport de son aire à son volume de 0,5 m²/m³ ou plus, au moins sur un tronçon de la partie creuse dans laquelle l'amine est oxydée à raison de 50 % (% en moles) ;
ii) la partie creuse présente une charge superficielle spécifique de 1 l/m²h à 40 l/m²h ; et/ou
iii) la partie creuse présente une charge volumique spécifique de 1000 l/m³h à 30 000 l/m³h ;
le fluide réactionnel étant envoyé dans la partie creuse avec un écoulement laminaire.

2. Procédé selon la revendication 1, **caractérisé en ce que** l'amine tertiaire est utilisée en tant qu'oxydant, de préférence selon un rapport en moles de 1:0,9 à 1:1,3, de préférence de 1:1 à 1:1,1, par rapport au peroxyde d'hydrogène.

3. Procédé selon la revendication 1 ou 2, **caractérisé en ce que** l'oxyde d'amine est le N-oxyde de N-méthyl-morpholine, et l'amine tertiaire est la N-méthyl-morpholine.

**4.** Procédé selon l'une des revendications 1 à 3, **caractérisé en ce qu'**on ajuste la vitesse d'écoulement du fluide réactionnel dans la partie creuse à un temps de séjour de l'amine tertiaire de 0,4 minute à 14 minutes, de préférence de 0,6 minute à 8 minutes.

**5.** Procédé selon l'une des revendications 1 à 4, **caractérisé en ce que** la vitesse d'écoulement du fluide réactionnel est de 0,1 m/min à 200 m/min, de préférence de 10 m/min à 160 m/min, tout spécialement de 10 m/min à 120 m/min.

**6.** Procédé selon l'une des revendications 1 à 5, **caractérisé en ce que** la température du fluide réactionnel dans la partie creuse est de 20 °C à 70 °C, de préférence de 40 °C à 65 °C, tout spécialement de 55 °C à 65 °C.

**7.** Procédé selon l'une des revendications 1 à 6, **caractérisé en ce que** la pression dans la partie creuse est de 1 bar à 200 bar, de préférence de 5 bar à 100 bar, tout spécialement de 50 bar à 100 bar.

**8.** Procédé selon l'une des revendications 1 à 7, **caractérisé en ce que** le diamètre intérieur d'une partie creuse, de préférence d'un tube, du réacteur est de 0,25 mm à 10 mm, de préférence de 0,5 mm à 6 mm, d'une manière particulièrement préférée de 0,8 mm à 4 mm.

**9.** Procédé selon l'une des revendications 1 à 8, **caractérisé en ce que** la longueur d'une partie creuse, de préférence d'un tube, du réacteur est de 0,5 m à 20 m, de préférence de 1 m à 10 m, en particulier de 2 m à 5 m.

**10.** Procédé selon l'une des revendications 1 à 9, **caractérisé en ce qu'**une paroi intérieure d'une partie creuse, de préférence d'un tube, du réacteur contient un ou plusieurs métaux, de préférence choisis parmi Fe, Cr, Ni, Mo, Co, Mn, Nb, Au, Pt, Ti ou des mélanges de ceux-ci, d'une manière particulièrement préférée la paroi intérieure d'une partie creuse, de préférence d'un tube, étant en acier austénitique.

**11.** Procédé selon l'une des revendications 1 à 10, **caractérisé en ce que** l'amine tertiaire est introduite dans la partie creuse à une concentration de 40 % à 100 %, de préférence de 50 à 99,5 %, en particulier de 60 à 99 %, d'une manière spécialement préférée de 70 % à 99 % (tous les pourcentages en % en volume).

**12.** Procédé selon l'une des revendications 1 à 11, **caractérisé en ce qu'**on introduit dans la partie creuse un oxydant, de préférence du peroxyde d'hydrogène, à une concentration de 5 % à 80 %, de préférence de 10 % à 70 %, en particulier de 20 % à 60 %, d'une manière spécialement préférée de 30 % à 55 % (tous les pourcentages en % en poids).

**13.** Procédé selon l'une des revendications 1 à 12, **caractérisé en ce que** le fluide réactionnel est envoyé dans la partie creuse avec un indice de Bodenstein supérieur à 10, de préférence de 20 à 200, selon la formule

$$Bo = u * L/D_{ax}$$

dans laquelle Bo est le nombre de Bodenstein, u est la vitesse d'écoulement du fluide réactionnel, L est la longueur de la partie creuse, de préférence du tube, du réacteur et $D_{ax}$ est le coefficient de dispersion axial du fluide réactionnel.

**14.** Procédé selon l'une des revendications 1 à 13, **caractérisé en ce qu'**on introduit dans la partie creuse du $CO_2$, de préférence en une quantité de 0,5 % en poids à 20 % en poids par rapport à l'amine tertiaire.

**15.** Procédé selon l'une des revendications 1 à 14, **caractérisé en ce qu'**on ajoute au fluide réactionnel un complexant de métaux, de préférence un phosphate, de préférence en une quantité de 0,01 % à 3 %, de préférence de 0,03 % à 2 %, d'une manière spécialement préférée de 0,05 % à 1,5 % (toutes en % en poids).

**16.** Procédé selon l'une des revendications 1 à 15, **caractérisé en ce que** l'oxydation de l'amine tertiaire est réalisée avec un oxydant, l'oxydant en excès étant dégradé ou éliminé.

**17.** Procédé selon l'une des revendications 1 à 16, **caractérisé en ce que** l'oxyde d'amine est porté à une concentration destinée à la dissolution de cellulose, et **en ce que**, en option, on y dissout de la cellulose supplémentaire, et, en option, on donne à la cellulose dissoute la forme d'un objet moulé continu, et on sépare par précipitation la cellulose des objets moulés.

**18.** Procédé selon l'une des revendications 1 à 17, **caractérisé en ce que** le fluide réactionnel est envoyé dans la partie creuse sous forme d'un écoulement présentant un nombre de Reynolds de 2300 ou moins, de préférence un nombre de Reynolds de 1000 ou moins, d'une manière particulièrement préférée un nombre de Reynolds de 1 à 100.

Fig. 1

Fig. 3

Fig. 2

Fig. 4

Fig. 5

Fig. 6

Fig. 7

Fig. 8

Fig. 9

Fig. 10

Fig. 12

Fig. 11

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2013030400 A
- US 2179181 A
- US 3447939 A
- DE 2557456 A1
- DD 246997 A1
- DE 3618352 A1
- EP 0307184 A2
- US 4247480 A
- US 4994614 A
- US 5216154 A
- DE 1221645 B
- WO 2010055034 A
- EP 0448924 B1
- DE 3504899 A1

### In der Beschreibung aufgeführte Nicht-Patentliteratur

- **NÉMETHNÉ et al.** *Materials Science and Engineering,* 2014, vol. 39 (2), 89-101
- **HOFFMANN et al.** *Combination of Anaerobic Treatment and Nutrient Removal of Wastewater,* 2010
- **HESSEL et al.** Micro Process Engineering, A comprehensive Handbook. Wiley-VCH, 2009
- **WINDSPERGER et al.** Praxis der biotechnologischen Abluftreinigung. Springer, 1996, 29